(19) Europäisches Patentamt
European Patent Office
Office européen des brevets

(11) EP 0 536 208 B1

(12) EUROPEAN PATENT SPECIFICATION

(45) Date of publication and mention of the grant of the patent:
**21.01.1998 Bulletin 1998/04**

(21) Application number: **91911663.2**

(22) Date of filing: **19.06.1991**

(51) Int Cl.[6]: **C07D 221/18**, C07D 401/04, C07D 401/06, A61K 31/44, A61K 31/495, A61K 31/535

(86) International application number:
**PCT/US91/04364**

(87) International publication number:
**WO 92/00281 (09.01.1992 Gazette 1992/02)**

# (54) 1,2-DIHYDRO-3H-DIBENZISOQUINOLINE-1,3-DIONE ANTICANCER AGENTS

1,2-DIHYDRO-3H-DIBENZOISOCHINOLIN-1,3-DIONE ALS ANTITUMORMITTEL

AGENTS ANTICANCEREUX A BASE DE 1,2-DIHYDRO-3H-DIBENZISOQUINOLINE-1,3-DIONES

(84) Designated Contracting States:
**AT BE CH DE DK ES FR GB GR IT LI LU NL SE**

(30) Priority: **26.06.1990 US 543596**

(43) Date of publication of application:
**14.04.1993 Bulletin 1993/15**

(73) Proprietor: **RESEARCH CORPORATION TECHNOLOGIES, INC.**
**Tucson Arizona 85711 (US)**

(72) Inventors:
- **ALBERTS, David, S.**
  **Tucson, AZ 85711 (US)**
- **DORR, Robert, T.**
  **Tucson, AZ 85718 (US)**
- **REMERS, William, A.**
  **Tucson, AZ 85718 (US)**
- **SAMI, Salah, M.**
  **Tucson, AZ 85719 (US)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:

| | |
|---|---|
| **FR-A- 2 392 978** | **US-A- 1 253 252** |
| **US-A- 3 940 398** | **US-A- 4 665 071** |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 536 208 B1

Printed by Jouve, 75001 PARIS (FR)

## Description

This invention is directed towards derivatives of azonafide having improved anti-tumor activity.

The search for compounds showing anti-tumor activity has, in recent years, included fused ring structures such as derivatives of anthracene, and heterocyclics such as isoquinoline and acridine. The first anthracene derivative to show promise was 2,2'-(9,10 anthracene-dimethylene)bis-(2-thiopseudourea)dihydrochloride, which unfortunately suffered from phototoxicity (U. S. Patent No. 3,190,795 and Carter, Cancer Chemother. Rep., 1, 153-163, 1968). See also, Frei, E. III, et al., Cancer Chemother Rep., 55, 91-97 (1971).

Furthermore, Brana, et al. in Cancer Chemother Pharmacol, 4, 61-66 (1980) and in Eur. J. Med., 16, 207-212 (1981) disclose 2- and 5- substituted benz[de]isoquinoline-1,3-diones having the formula:

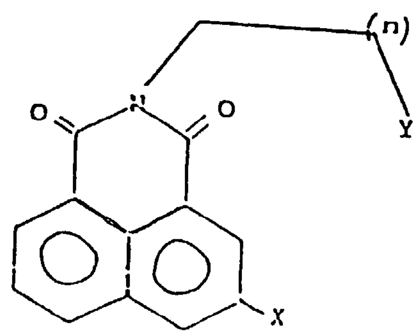

wherein X is H, $NO_2$, $NH_2$, Cl, OH, $NHCO_2Et$, $OCH_3$, $NHCOCH_3$ or t-Bu and Y is a disubstituted amine, OH, $OCH_3$, $CH(CH_3)_2$, SH or $NHCOCH_3$ and n is an integer ranging from zero to three. It is alleged that the compounds therein inhibit Hela Cells.

Miller, et al. in U. S. Patent No. 4,108,896 discloses compounds having the formula:

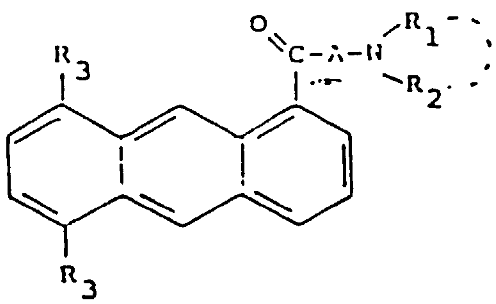

wherein A is a straight or branched alkylene chain having from 1 to 6 carbon atoms; $R_1$ and $R_2$ are each selected from the group consisting of hydrogen, lower alkyl having from 1 to 6 carbon atoms, cycloalkyl having from 3 to 6 carbon atoms, alkenyl having from 3 to 6 carbon atoms in which the unsaturation is in a position other than in the 1-position of the alkenyl group, or $R_1$ and $R_2$ taken together with the nitrogen atom to which they are attached, represent the pyrrolidinyl, piperidine or morpholine radical; $R_3$ is selected from the group consisting of hydrogen and the radical,

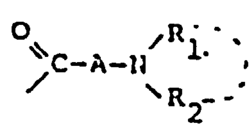

with the proviso that one and only one such R group is hydrogen.

The compounds are disclosed as having use as antiviral agents.

However, the teachings of the references discussed hereinabove are limited to the anthracene and isoquinoline derivatives discussed therein. None of these references suggest that the dibenzisoquinoline 1,3-dienes of the present

invention would be useful and effective as anti-tumor agents.

Amonafide (NSC 308847) is an isoquinoline dione derivative having anti-tumor activity. More specifically, amonafide, amino-N-dimethylaminoethylbenz[de]isoquinoline, has undergone extensive tests for its anti-tumor activity. The National Cancer Institute prepared and distributed a brochure summarizing the anti-tumor activity of amonafide in 1984. Although the level of activity found for amonafide was and continues to be of high interest, this material does have significant deficiencies which indicate the continuing need for agents with improved properties. In the first place, amonafide has produced substantial myelotoxicity leading to some deaths in patients receiving five daily doses of the drug. In addition, this report showed that amonafide had only moderate activity in leukemia models in mice. Also, it showed that amonafide has no activity in human tumor xenographs in mice with colon, lung and mammary cancers. Thus, while amonafide showed significant activity, it does not have a substantially broad spectrum of activity in murine tumor models.

Another group has shown that amonafide or nafidimide have poor activity when tested in primary human solid tumors in vitro. See, Ajani, J. A. et al., Invest New Drugs, 6, 79-83 (1988).

FR-A-2 392 978 and US-A-4,665,071 disclose compounds which differ from the present compounds in that they are substituted naphthalene dicarboxylic acid imide derivatives but no anthracene 1,9-dicarboxylic acid imides. Furthermore, the anthracene 1,9-dicarboxylic acid imide system has only been disclosed to be useful for colouring matters, cf. US-A-1,253,252.

In contrast, we have found that compounds based on anthracene instead of naphthalene show surprising anti-tumor activity.

The present invention is directed to 1,2-dihydro-3H-dibenzisoquinoline-1,3-dione derivatives which exhibit anti-tumor activity and are useful as anti-cancer agents.

More particularly, the present invention is directed to compounds of the formula:

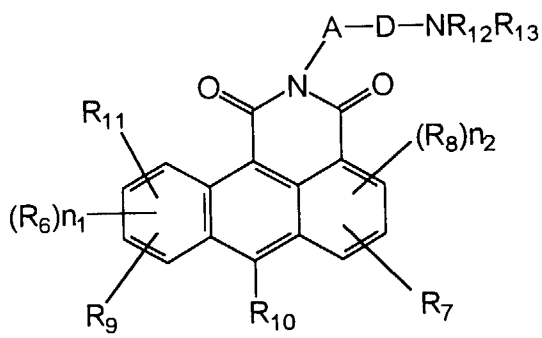

I

wherein

$R_8$, $R_6$ and $R_{10}$ are independently hydrogen, $C_1$-$C_6$ alkyl, aryl, $C_2$-$C_7$ alkanoyl, formyl, halogen, nitro, $NR_2R_3$, $OR_1$, or $SR_1$, hydroxy, methoxy, cyano, $CO_2H$, $SO_2NR_1R_2$, or $CONR_1R_2$; $R_1$, $R_2$ and $R_3$ are independently hydrogen, $C_1$-$C_6$ alkyl, aryl-($C_1$-$C_6$-alkyl), aryl, formyl or $C_2$-$C_7$ alkanoyl;
$R_9$, $R_{11}$, $R_{10}$ and $R_7$ are independently hydrogen, or $C_1$-$C_6$ alkyl or
$R_9$ and $R_{11}$ taken together with the carbon atoms to which they are attached form a phenyl group or
$R_9$ and $R_{10}$ taken together with the carbon atoms to which they are attached form a phenyl group or
$R_7$ and $R_{10}$ taken together with the carbon atoms to which they are attached form a phenyl group;
A is $(CR_4R_5)n_3$, $C_3$-$C_{10}$ cycloalkyl or aryl or a chemical bond; each $R_4$ and $R_5$ are independently hydrogen or $C_1$-$C_6$ alkyl;
$R_{12}$ and $R_{13}$ are independently hydrogen, or $C_1$-$C_6$ alkyl which is unsubstituted or substituted with hydroxy, mercapto, $C_1$-$C_6$ alkoxy, $C_2$-$C_7$ alkylcarbonyloxy, carboxy, or carbloweralkoxy or $R_{12}$ and $R_{13}$ taken together with the nitrogen to which they are attached form a 3-6-membered heterocyclic ring;
D is a chemical bond, or taken together with $NR_{12}$ form a 5 or 6-membered heterocyclic ring;
$n_1$ and $n_2$ are independently 0, 1 or 2; and
$n_3$ is 0, 1, 2, 3, 4 or 5.

These compounds are useful in treating cancer in animals, including mammals by administering to said animals, an effective anti-tumor dose of said compounds.

As indicated hereinabove, the present invention is directed to 1,2-dihydro-3H-dibenz(deh)isoquinoline-1,3-dione

derivatives. Since the ring structure may have substituents at various positions, to aid in the understanding of the various derivatives, the nomenclature with respect to the dibenzisoquinoline structure is as indicated hereinbelow:

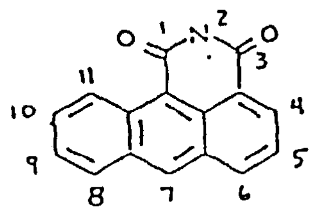

As used herein, the term "alkyl", when used alone or in combination, consists of a carbon chain containing from one to six carbon atoms. The alkyl groups may be a straight chain or a branched chain. It includes such groups as methyl, ethyl, propyl, isopropyl, n-butyl, see-butyl, iso-butyl, t-butyl, n-pentyl, amyl, n-hexyl, and the like. The preferred alkyl group is methyl.

The term "aryl", when used alone or in combination, consists of an aromatic monocyclic or bicyclic structure having 6 to 10 ring carbon atoms and up to a total of 15 total carbon atoms. It includes such structures as phenyl, -naphthyl or (β-naphthyl. The preferred aryl group is phenyl.

The term "aryl lower alkyl", is an aryl group attached to the dibenzisoquinoline ring through an alkylene group, such as methylene, ethylene, propylene and the like. Examples include benzyl, phenethyl and the like. The preferred aryl lower alkyl group is benzyl.

"Alkylene", as used herein, whether alone or in combination, is an alkyl group attached to the principal chain of the compound of the present invention through two carbon linkages. This group may be straight chained or branched. It includes such groups as methylene ($-CH_2-$), ethylene ($-CH_2-CH_2-$), propylene ($-CH_2-CH_2-CH_2-$), isopropylene

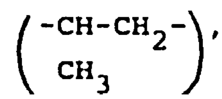

isobutylene, butylene, sec-butylene, and the like.

As used herein, the term "alkanoyl" is an alkyl group substituted by an oxo group. The oxo group can be substituted at any carbon atom, but is preferred at the 1-position, i.e., the carbon atoms directly attached to the dibenzisoquinoline ring structure. This group includes acetyl, propanoyl, butanoyl, and the like. The preferred group is acetyl.

Halogen, as used herein, refers to fluorine, chlorine, bromine or iodine.

The term "lower cycloalkyl" refers to a monocyclic alkyl group containing from 3 to 6 ring carbon atoms and up to a total of 10 carbon atoms. This group includes cyclopropyl, cyclobutyl, cyclopentyl, cyclohexyl and the like. The preferred cycloalkyl groups are cyclopentyl and cyclohexyl.

The heterocyclic rings as defined herein are 3-6 membered rings containing at least one oxygen, sulfur and nitrogen ring atom and up to a total of 4 ring heteroatoms. It is preferred, however, that there may be one or two ring heteroatoms. Especially preferred is one ring heteroatom. The preferred heteroatom is nitrogen. The heterocyclic ring may be completely saturated or partially unsaturated or may be heteroaromatic. It is preferred that the heterocyclic ring contain 5 or 6 ring atoms. Examples include thiophene, furan, pyran, pyrrole, imidazole, pyrazole, pyridine, pyrazine, pyrimidine, pyridazine, isothiazole, furazan, isoxazole, imidazolidine, imidazoline, pyrazolidine, piperidine, morpholine, pyrrolidine, tetrahydrofuran, tetrazole, and the like. The preferred heterocyclic groups are piperidine, pyrrolidine, piperazine, or imidazole, pyridyl, or aziridinyl. The especially preferred heterocyclic groups are piperidine and pyrrolidine.

As indicated in the above formula, the side chain "$A-D-NR_{12}R_{13}$" is attached to the nitrogen at the 2-position of the dibenzisoquinoline-1,3-diones. This group can be a straight chain, such as $(CH_2)n_3NR_{12}R_{13}$, wherein $n_3$ is 1-5 and $R_{12}$ and $R_{13}$ are each lower alkyl or hydrogen. Alternatively, $R_{12}$ and $R_{13}$ may with the nitrogen to which they are attached from a 3 to 6 membered ring, such as pyrrolidine or piperidine.

In addition, the $NR_{12}$ group together with D may form a 5 or 6 membered nitrogen heterocyclic ring, such as a piperidine or pyrrolidine, e.g.,

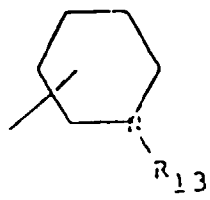

or

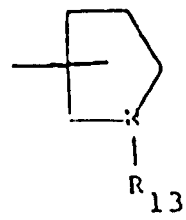

wherein $R_{13}$ is as defined hereinabove.

The groups $R_9$ and $R_{11}$ may together form an aryl ring. For example, if $R_9$ and $R_{11}$ form a phenyl ring, the compound of Formula I becomes:

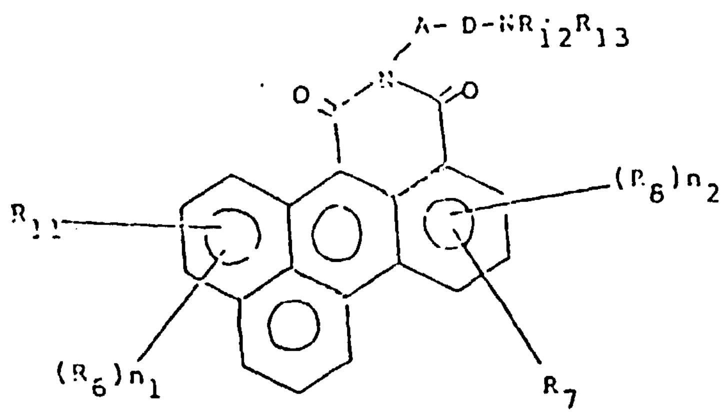

Alternatively, $R_{10}$ and $R_9$ may together form an aryl ring, e.g., phenyl ring. Then the compound of Formula I will become:

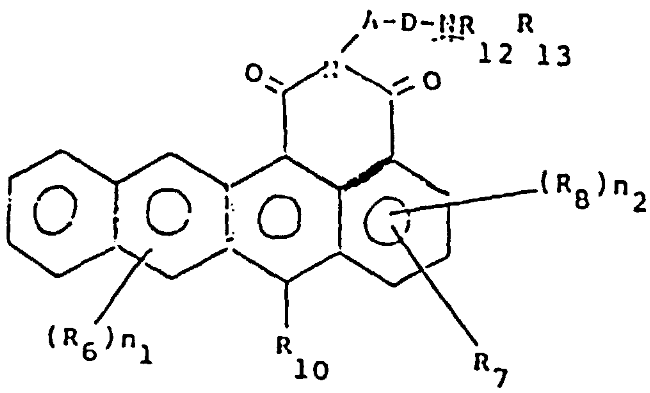

Furthermore, when $R_7$ and $R_{10}$ taken together with the carbon atoms to which they are attached form an aryl group, such as phenyl, the compound of Formula I becomes:

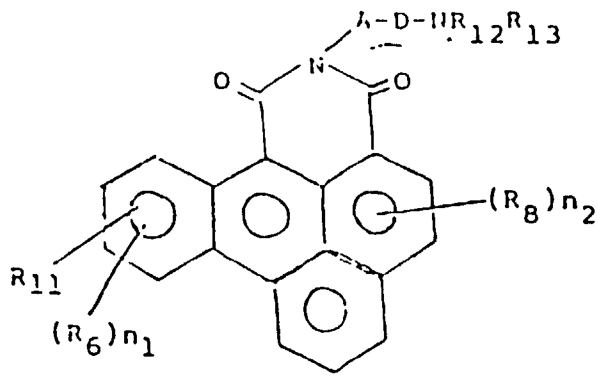

In all of these structures hereinabove, $R_{11}$, $R_6$, $R_9$, $R_{10}$, $R_7$, $R_8$, A, D, $R_{12}$, $R_{13}$, $n_1$ and $n_2$ are as defined hereinabove.

A preferred embodiment of the present invention has the formula:

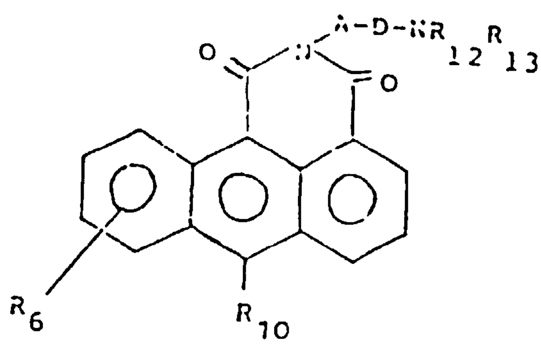

wherein $R_6$, A, D, $R_{12}$ and $R_{13}$ are as defined hereinabove. It is preferred that $R_6$ is hydrogen, amino, chloro or nitro. Moreover, it is preferred that $R_6$ is substituted on the 8-, 9, 10- or 11-position of the dibenzisoquinoline-1,3-dione of the present invention.

The preferred $R_1$, $R_2$ and $R_3$ groups are hydrogen or methyl. Therefore, it is preferred that $NR_2R_3$, $OR_1$, and $SR_1$ groups are amino, hydroxy or mercapto.

it is preferred that A is alkylene containing from 1-4 carbon atoms, aryl or a chemical bond. It is especially preferred that the alkylene group is of the formula $(CH_2)n_3$, wherein $n_3$ is 2-3. The preferred aryl group is phenyl.

The most preferred $R_{12}$ and $R_{13}$ groups are lower alkyl or lower alkyl substituted with hydroxy. When $NR_{12}$ does not form a ring with D, it is preferred that the $R_{12}$ and $R_{13}$ groups be the same. The most preferred $R_{12}$ and $R_{13}$ groups are methyl or $CH_2CH_2OH$.

It is preferred that $R_{10}$ is hydrogen, methyl, methoxy, chloro or hydroxy.

An especially preferred embodiment of the present invention has the formula:

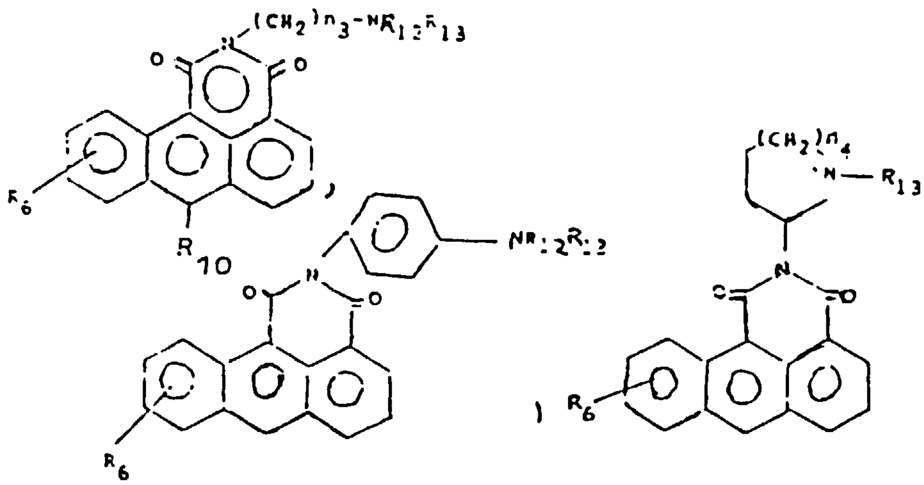

wherein $R_{12}$, $R_{13}$, $n_3$, $R_6$ and $R_{10}$ are as defined hereinabove, and $n_4$ is 0 or 1.

The compounds of the present invention can be prepared by art recognized techniques. More specifically, the compounds of this invention can be prepared by the condensation of anthracene-1,9-dicarboxylic anhydride of formula II with an amine of formula $NH_2$-A-D-$NR_{12}R_{13}$ (III) as indicated hereinbelow:

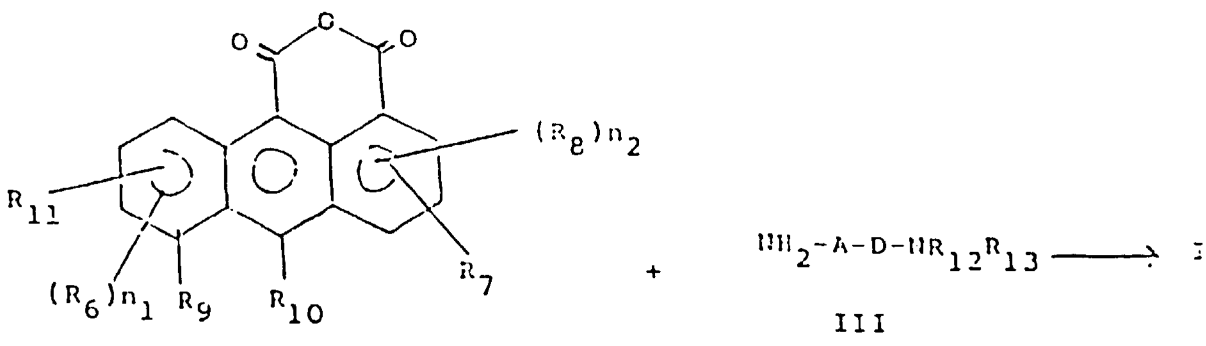

III

II

In the above equation, $R_1$, $R_2$, $R_3$, $R_4$, $R_5$, $R_6$, $R_7$, $R_8$, $R_9$, $R_{10}$, $R_{11}$, $R_{12}$, $R_{13}$, A, D, $n_1$, $n_2$ and $n_3$ are as defined hereinabove.

The reaction is carried out in inert solvents which are inert to both reactants and products and will dissolve both reactants, e.g., toluene, benzene, petroleum ether, hexanes, methylene chloride, chloroform, carbon tetrachloride alcohol, e.g., methanol, ethanol, and the like. The reaction can be effected at room temperature up to the reflux temperature of the solvent. The preferred solvent is toluene, and it is preferred that the reaction be run at reflux temperatures at a time sufficient for the condensation to occur, e.g., 2-24 hours.

When $R_{10}$ is halogen, the other groups representative of $R_{10}$ can be prepared by nucleophilic displacement of said halogen at $R_{10}$ by strong nucleophiles such as hydroxide and methoxide.

If there are reactive groups on $R_8$ or $R_6$, such as $NH_2$, OH, or $SR_1$, they can be protected by blocking groups known in the art. Many of these blocking groups are described in "Protective Groups in Organic Synthesis" by T.W. Greene, John Wiley and Sons, New York, New York, 1981, the contents of which are incorporated herein by reference. For example, when $R_8$ or $R_6$ is $NH_2$, it can be protected by such groups as N-formyl, and N-acetyl, and the like.

Alternatively, these reactive groups can be placed on the rings after the condensation takes place. The following scheme is exemplary:

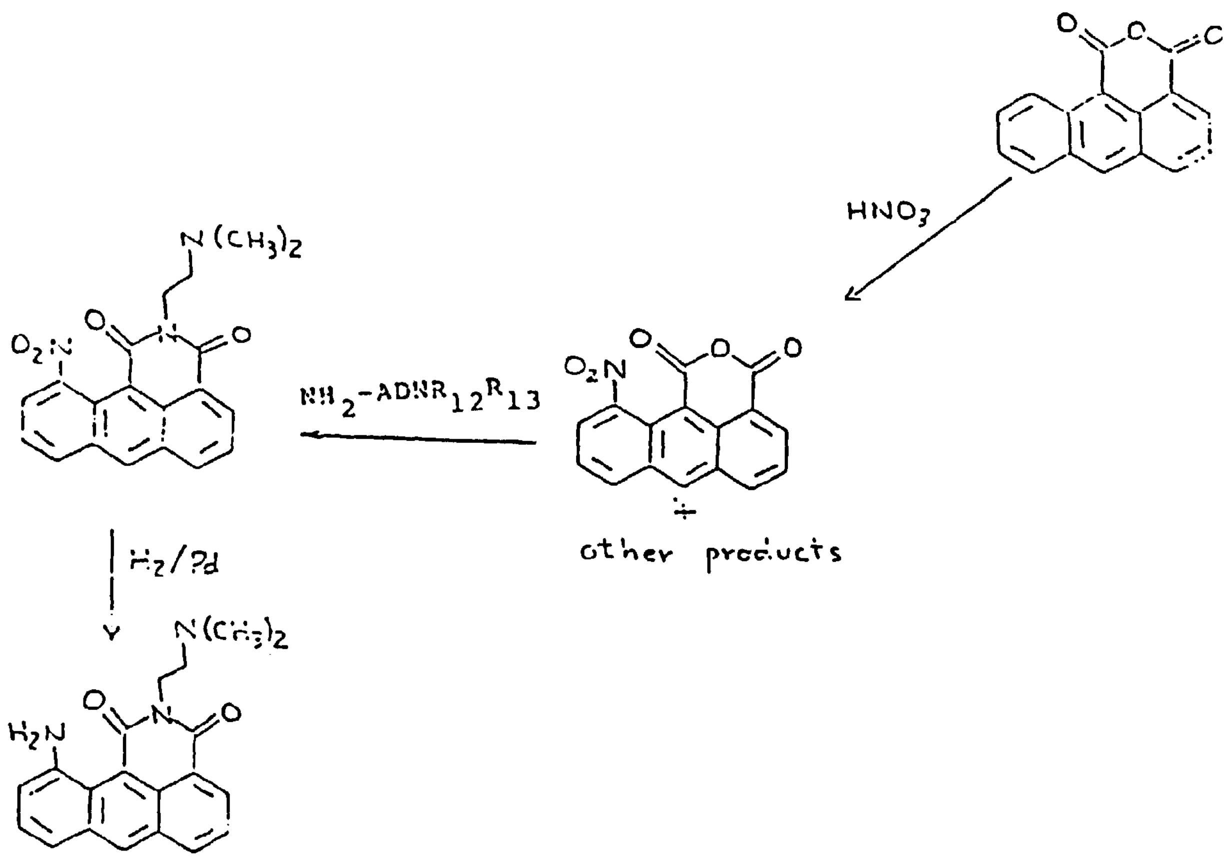


In this scheme, A, D, $R_{12}$ and $R_{13}$ are as defined hereinabove.

The anthracene-1,9-dicarboxlic anhydride (II A) is nitrated with nitric acid, which is then condensed with the amine to form the nitrated dibenz-isoquinoline-1,3-dione derivative. The nitrated compound is then reduced by a reducing agent, such as $H_2$/Pd, or $H_2$/Pt and the like, to form the corresponding amine.

As another example, a compound of Formula I, wherein A D, $R_1$, $R_2$, $R_3$, $R_4$, $R_5$, $R_7$, $R_8$, $R_9$, $R_{10}$, $R_{11}$, $n_1$, $n_2$ and $n_3$ are as defined hereinabove and $R_6$ is $SO_2NH_2$ can be formed as follows:

A compound of Formula I, wherein A D $R_8$, $R_{10}$, $R_1$, $R_2$, $R_3$, $R_9$, $R_{11}$, $R_7$, $R_4$, $R_5$, $R_{12}$ and $R_{13}$ are as defined hereinabove and $R_6$ is hydrogen, is reacted with chlorosulfonic acid ($ClSO_3H$) followed by simple addition by $NR_{12}R_{13}$ to form the above said compound.

The anthracene 1,9-dicarboxylic anhydride (II) can also be prepared by art recognized techniques.

An examplary procedure is indicated hereinbelow:

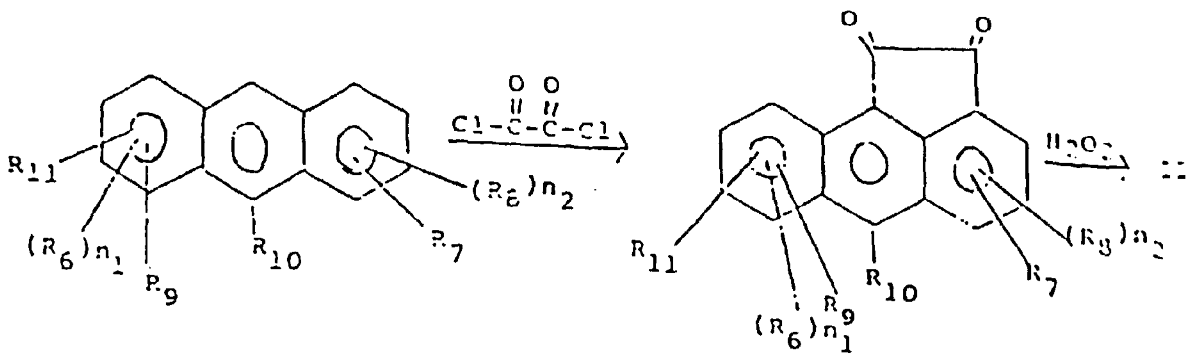

IV V

In the above procedure, $R_{11}$, $R_6$, $R_9$, $R_{10}$, $R_9$, $R_8$, $n_1$ and $n_2$ are as defined hereinabove.

The anthracene derivative II is prepared by treating an anthracene with oxalyl chloride, followed by oxidation with hydrogen peroxide in accordance with the procedure described by E.D. Bergmann and R. Ikan, J. Org. Chem., 23, 907 (1958).

5-substituted compounds of formula I can also be prepared by synthesis from the corresponding 5-substituted indanones. For example, 5-nitroindanone, treated with sodium borohydride followed by triphenylphosphine dibromide, furnishes l-bromo-5-nitroindan, which is converted into a Grignard reagent by magnesium in ether. Addition of 2-cyclohexenone and cuprous iodide gives 1-(cyclohexanon-3-yl)-5-nitroindan. Conversion of this product into its hydroxymethylene derivative by ethyl formate and sodium ethoxide, followed by cyclization in the presence of polyphosphoric acid affords 7-nitro-4-oxo-1,2,3,4,8b,11a-hexahydrocyclopentanoanthracene, which gives 3-nitro-1, 9-cyclopentanoanthracene on reduction with sodium borohydride followed by treatment with dichlorodicyanobenzoquinone. Oxidation of this product with sodium dichromate, followed by acidification furnishes 5-nitro-1,9-dicarboxylic acid anhydride, which gives the desired 5-nitro compound of formula I on treatment with an amine such as dimethylethylenediamine. The nitro group of this compound can be reduced catalytically to an amine using techniques known to one skilled in the art, such as $H_2/Pd$ or $H_2/Pt$. This amine can be acetylated using an acetyling reagent, such as acetic anhydride. These transformations are exemplified hereinbelow:

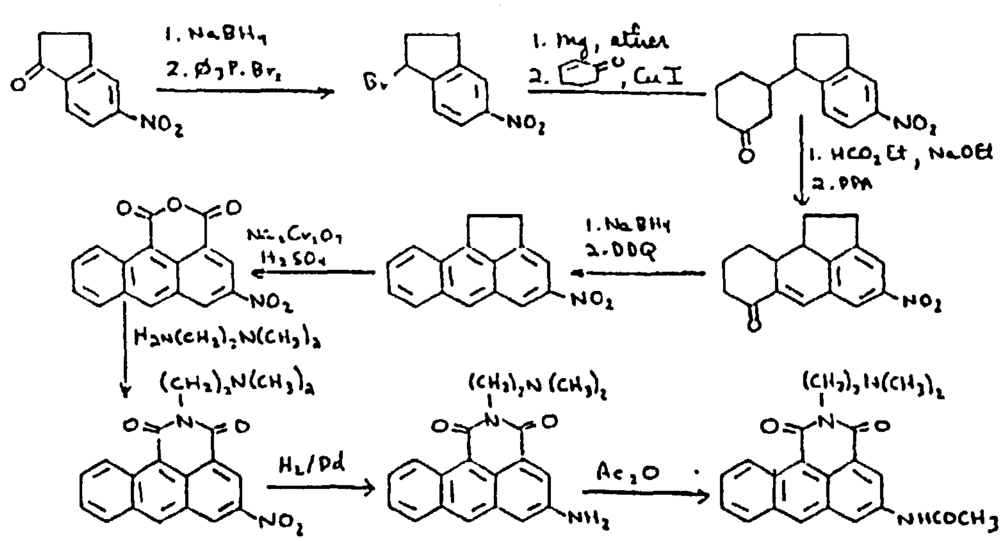


The compounds of the invention containing basic nitrogen form salts with acids, both organic and inorganic acids. Of particular value are salts with pharmaceutically-acceptable acids especially in dosage forms predicated on aqueous systems where the enhanced water solubility of the salts is most advantageous. Salts formed with pharmaceutically unacceptable acids are also useful in the isolation and purification of the basic nitrogen-containng present new compounds. Salts include those formed with hydrochloric, sulfuric, nitric, perchloric, benzenesulfonic, toluenesulfonic, phosphoric, acetic, malic, malonic, tartaric and similar such acids.

The compounds of the present invention can be administered to the host in a variety of forms adapted to the chosen route of administration, i.e., orally, intravenously, intramuscularly or subcutaneously.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium sterate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. in all cases the form must be sterile and must be fluid to the extent that easy syringability exists. It may be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The following examples further illustrate the invention. These examples are provided solely for illustrative purposes; thus, the present invention should not be limited thereto.

<u>EXAMPLE 1</u>

<u>Preparation of Anthracene-1,9-Dicarboxylic Acid Anhydride</u>

A suspension of 6.5 g of anthracene-1,9-dicarboxylic acid (E.D. Bergmann and R. Ikan, <u>J. Org. Chem.</u>, <u>23</u>, 907 (1958)) in 100 ml of acetic anhydride was heated at reflux for 3 hours. The mixture was cooled and the orange precipitate was collected by filtration, washed with ether and dried in air to give 5.1 g (68%) of the title compound. Recrystallization

from dimethylsulfoxide or toluene gave orange plates with melting point 289-290°C.

EXAMPLE 2

2-[2'-(dimethylamino)ethyl]-1,2-dihydro-3H-dibenz(deh)isoquinoline-1,3-dione (1)

A suspension of 248 mg (1 mmol) of anthracene-1,9-dicarboxylic acid anhydride in 25 ml of toluene was treated with 106 mg (1.2 mmol) of N,N-dimethylethylenediamine. The mixture was refluxed for 4 hours. The clear yellow reaction solution was concentrated under reduced pressure to an oily residue which was isolated on a silica gel column using a mixture of chloroform-methanol (9.5-0.5 or 9:1) as a solvent to give 245 mg (77%) of the title compound, crystallized from toluene, m.p 126-128°C and providing the following analysis.

$^1H$ NMR ($d_6$DMSO, TS), δ values in ppm.
2.3 (s,6,N-$CH_3$), 2.4-2.65 (t,2,N-$CH_2$), 4.0-4.25 (t,2,CON-$CH_2$), 7.55-7.9 (m,3,protons 5 + 9 + 10), 8.05-8.20 (d, 1,H-8), 8.3-8.5 (t<<d over d>>,2,H-4 + H-6), 8.9 (s,1,H-7), 9.6-9.8 (d,1,H-11).

EXAMPLE 3

2-[2'-(N-pyrrolidino)ethyl]-1,2-dihydro-3H-dibenz(deh)isoquinoline-1,3-dione (8)

A suspension of 500 mg (2.02 mmol) of anthracene-1,9-dicarboxylic anhydride in 10 ml toluene was treated with 250 mg (2.20 mmol) of l-(2-aminoethyl)-pyrrolidine. The mixture was refluxed overnight. The clear reaction solution was separated from resins by decantation. It was then allowed to cool to room temperature. The crystalline yellow material deposited (640 mg. 92%) was collected and recrystallized from hexane-toluene 1:1 to give yellow crystals of the title compound having a m.p. of 162-164°C and providing the following analysis:

$^1H$ NMR ($CDCl_3$,TS), δ values in ppm.
1.65-1.95 (m,4,-$CH_2$-), 2.5-3.0 (m,6,N-$CH_2$), 4.35-4.55 (t,2,CON-$CH_2$), 7.53-7.9 (m,3,H-5 + H-9 + H-10), 8.0-8.1 (d,1,H-8), 8.23-8.33 (d,1, H-4), 8.65-8.75 (s over d,2,H-6 + H-7), 9.9-10.0 (d,1,H-11).

EXAMPLE 4

2-[2'-(N-piperidino)ethyl]-1,2-dihydro-3H-dibenz(deh)isoquinoline-1,3-dione (7)

A suspension of 500 mg (2.02 mmol) of anthracene-1,9-dicarboxylic anhydride in 10 ml toluene was treated with 283 mg (2.21 mmol) of 1-(2-aminoethyl)piperidine. The mixture was refluxed overnight under nitrogen. The clear reaction solution was separated from tarry material by decantation. It was then allowed to cool to room temperature. The dark yellow solid that precipitated (715 mg, 99%) was collected and crystallized from a mixture of hexane-toluene 1: 1, affording yellow crystals of m.p. 171-173°C and providing the following analysis:

$^1H$ NMR ($CDCl_3$, TS), δ values in ppm.
1.1-1.8 (m,6,-$CH_2$-), 2.5-2.9 (m,6,N-$CH_2$), 4.35-4.55 (t,2,CON-$CH_2$), 7.55-7.90 (m,3,H-5 + H-9 + H-10), 8.05-8.15 (d,1,H-8), 8.25-8.35 (d,1,H-4), 8.65-8.75 (s over d, 2, H-6 + H-7), 9.9-10.0 (d,2,H-11).

EXAMPLE 5

2-(1'-ethyl-3'piperidinyl)-1,2-dihydro-3H-dibenz(deh)isoquinoline-1,3-dione (9)

A suspension of 600 mg (2.42 mmol) of anthracene-1,9-dicarboxylic acid anhydride in 10 ml toluene was treated with 343 mg (2.68 mmol) of 3-amino-1-ethyl piperidine. The mixture was refluxed under nitrogen overnight. The clear reaction solution was separated from tarry material by decantation. The toluene was evaporated to give 800 mg (92%) of light brown solid, which was crystallized from a mixture of hexane-toluene (2:1) as buff crystals of the title compound, having melting point 163-165°C and providing the following analysis:

$^1H$ NMR ($CDCl_3$, TS), δ values in ppm
1.05-1.20 (t,3,$CH_3$), 1.3-2.2 (m,4,-$CH_2$-), 2.25-2.75 (m,4,N-$CH_2$ endocyclic), 2.9-3.1 (m,2,N-$CH_2$ exocyclic), 5.2-5.62 (m,1,CON-CH), 7.5-7.9 (m,3,H-5 + H-9 + H-10), 8.0-8.10 (d,1,H-8), 8.25-8.35 (d,1,H-4), 8.65-8.75 (s over d,2,H-6 + H-7), 9.85-9.95 (d,1,H-11).

EXAMPLE 6

2-[3'-(Diethanolamino)propylamino]-1,2-dihydro-3H-dibenz(deh)isoquinoline-1,3-dione (12)

A suspension of 248 mg (1 mmol) of anthracene-1,9-dicarboxylic anhydride in 45 ml of dry toluene was treated with 194 mg (1.2 mmole) of N-(3-aminopropyl)diethanolamine in 1 ml of absolute ethanol The mixture was refluxed under nitrogen for 7 hours. The solvent was evaporated and the residue was isolated on a silica gel column using a mixture of chloroform-methanol (8:2) as a solvent to give 311 mg (79%) of the title compound which was crystallized from toluene into yellow needles of melting point 139-141°C and providing the following analysis:

$H^1$ NMR ($CDCl_3$, TS), $\delta$ values in ppm
1.8-2.1 (quintuplet,2,-$CH_2$-), 2.65-2.8 (m,6,N-$CH_2$), 3.68-3.78 (t,4,$CH_2$-OH), 3.0-3.3 (br,2,OH), 4.2-4.4 (t,2,CON-$CH_2$), 7.5-7.85 (m,3,H-5 + H-9 + H-10), 7.95-8.05 (d,1,H-8), 8.15-8.25 (d,1,H-4), 8.6-8.7 (s over d,2,H-6 + H-7), 9.75-9.85 (d,1,H-11).

EXAMPLE 7

2-[3'-(dimethylamino)propyl]-1,2-dihydro-3H-dibenz(deh)isoquinoline-1,3-dione (11)

A suspension of 600 mg (2.42 mmol) of anthracene-1,9-dicarboxylic acid anhydride in 15 ml toluene was treated with 280 mg (2.75 mmol) of 3-dimethylaminopropylamine. The mixture was refluxed under nitrogen overnight. The clear solution was separated from tarry material by decantation. Evaporation of the solvent gave 715 mg (89%) of the title compound which crystallized from a mixture of hexane-toluene (2:1) as yellow needles of melting point 111-113°C and providing the following analysis:

$^1H$ NMR ($CDCl_3$, TS), $\delta$ values in ppm
1.9-2.15 (quintuplet, 2, -$CH_2$-), 2.3 (s,6,N-$CH_3$), 2.4-2.6 (t,2,N-$CH_2$), 4.2-4.4 (t,2,CON-$CH_2$), 7.48-7.85 (m,3,H-5 + H-9 + H-10), 7.95-8.05 (d,1,H-8), 8.2-8.3 (d,1,H-4), 8.60-8.70 (s over d,2,H-6 + H-7), 9.85-9.995 (d,1,H-11).

EXAMPLE 8

2-(4'-dimethylaminophenyl)-1,2-dihydro-3H-dibenz(deh)isoquinoline-1,3-dione (10)

A suspension of 300 mg (1.21 mmole) of anthracene-1,9-dicarboxylic anhydride in 40 ml of absolute ethanol was treated with a solution of 494 mg (3.63 mmole) of N,N-dimethyl-p-phenylenediamine in 10 ml of absolute ethanol. After refluxing the mixture under nitrogen for 24 hours, 20 ml of dry toluene was added and the mixture was refluxed for another 72 hours. The insoluble yellow solid (340 mg) was filtered off and dried in air. It was boiled with 100 ml of dioxane and the insoluble material (110 mg) which represents unreacted anhydride was filtered off. The filtrate, upon evaporation, gave 230 mg (82% based on reacted amount) of the title compound which was crystallized from dioxane into yellow needles having a melting point of 332-334°C and providing the following analysis:

$^1H$ NMR ($d_6$DMSO, TS), $\delta$ values in ppm.

3.18 (s,6,N-$CH_3$), 7.37-7.43 (t,1,H-9), 7.47-7.61 (m<<d over t>>,4,H-5 + H-10 + H-3' + H-5'), 7.69-7.72 (d,2,H-2' + H-6'), 7.89-7.92 (d,1,H-8), 8-18-8.22 (d,1,H-4), 8.41-8.44 (d,1,H-6), 8.65 (s,1,H-7), 9.50-9.56 (d,1,H-11).

EXAMPLE 9

2-[2'-(dimethylamino)ethyl]-1,2-dihydro-8-nitro-3H-dibenz(deh)isoquinoline-1,3-dione (13) and 2-[2-(dimethylamino) ethyl]-1,2-dihydro-11-nitro-2H-dibenz(deh)isoquinoline-1,3 -dione (2)

A stirred solution of 416 mg (1.68 mmol) of anthracene-1,9-dicarboxylic acid anhydride in 25 ml of concentrated sulfuric acid was treated at -10 to -12°C with a solution of 155 mg of 70% nitric acid (1.7 mmol) in 1 ml of concentrated sulfuric acid. Stirring was continued for 15 minutes after the addition was complete and then the mixture was poured over ice water. The resulting yellow precipitate, a mixture of isomeric mononitro derivatives, was washed well with water and dried in air. It was used directly in the next step.

A suspension of 570 mg (1.95 mmol) of a mixture of mononitro derivatives in 50 ml of dry toluene was treated with a solution of 206 mg (2.35 mmol) of N,N-dimethylethylenediamine in 15 ml of dry ethanol. The mixture was heated at

reflux for 4 hours, during which time a clear brownish yellow solution formed. After evaporation of the solvent, the solid residue was separated into its components by chromatography on a silica gel column using chloroform-acetone (1:1) as solvent. Concentration of the first yellow fraction gave 247 mg (35%) of 2-[2'-(dimethylamino)ethyl]-1,2-dihydro-11-nitro-3H-dibenz(dehlisoquinoline-1,3-dione, which was crystallized from toluene to give yellow flakes with melting point 238-240°C and providing the following analysis:

$^1$H NMR ($d_6$DMSO, TS), δ values in ppm.

2.99 (s,6,$NCH_3$), 3.52-3.57 (t,2,$NCH_2$), 4,49-4.53 (t,2,$CONCH_2$), 7.79-7.86 (t,1,H-5), 7.92-7.98 (t,1,H-9), 8.47-8.50 (d,1,H-4), 8.59-8.67 [doublet over doublet (appears as triplet), 2,H-6 + H-8], 8.75-8.77 (d,1,H-10), 9.32 (s,1,H-7).

Concentration of the second yellow fraction gave 181 mg (26%) of 2-[2'-(dimethylamino)ethyl)-1,2-dihydro-8-nitro-3H-dibenz(deh)isoquinoline-1,3-dione, which was crystallized from hexane-toluene (1:1) into brownish-yellow cubes with melting point 210-212°C and providing the following analysis:

$^1$H NMR ($CDCl_3$, TS), δ values in ppm.

2.40 (s,6,$NCH_3$), 2.70-2.77 (t,2,$NCH_2$), 4.39-4.45 (t,2,CON-$CH_2$), 7.77-7.86 (m,2,H-5 + H-10), 8.27-8.30 (d,1,H-4), 8.35-8.39 (d,1,H-6), 8.75-8.80 (d,1,H-9), 9.42 (s,1,H-7), 10.34-10.38 (d,1,H-11).

EXAMPLE 10

8-Amino-2-[2'-(dimethylamino)ethyl]-1,2-dihydro-3H-dibenz-(deh)isoquinoline-1,3-dione (14)

A solution of 84 mg of 2-[2'-(dimethylamino)ethyl]-8-nitro-1,2-dihydro-3H-dibenz-(deh)isoquinoline-1,3-dione in 100 ml of absolute ethanol was treated with 10 mg of palladium-on-carbon catalyst and shaken with hydrogen at 42 p.s.i. for 5 hours. The mixture was filtered and the filtrate was evaporated to give 77 mg (99.9%) of the title compound as a brown solid that melted partially at 165-168°C and completely at 200-202°C (melting point of the dihydrochloride salt was above 300°C).

EXAMPLE 11

2-[2'-(dimethylamino)ethyl]1,2-dihydro-6-ethyl-3H-dibenz(deh)isoquinoline-1,3-dione (15)

A solution of 500 mg of 2-[2'-(dimethylamino)ethyl]-1,2-dihydro-3H-dibenz(deh)isoquinoline-1,3-dione in 30 ml of dry tetrahydrofuran was treated with 4 ml of a 2M solution of ethyl magnesium bromide in tetrahydrofuran. The mixture was stirred overnight and then poured into saturated ammonium chloride solution. The two layers were separated and the aqueous layer was extracted with chloroform. The combined organic layers were dried over sodium sulfate and then evaporated to give an oily residue that was separated into its components by preparative thin-layer chromatography on silica gel with acetone-toluene (2:8) as solvent to give starting material (94 mg), a polar brown oil containing 3 components (268 mg), and the title compound (least polar) (118 mg, 27% based on converted starting material) as a yellow solid. The title compound gave upon recrystallization from hexane-toluene (3:1) yellow needles having a melting point of 148-150°C and providing the following analysis:

$^1$H NMR ($CDCl_3$, TS), δ values in ppm

1.37-1.43 (t,3,$CH_3$), 2.42 (s,6,N-$CH_3$), 2.69-2.75 (t,2,N-$CH_2$), 3.44-3.53 (q,2,$CH_2$), 4.39-4.44 (t,2,CON-$CH_2$), 7.46-7.49 (d,1,H-5), 7.53-7.59 (t,1,H-9), 7.72-7.79 (t,1,H-10), 7.98-8.02 (d,1,H-8), 8.10-8.13 (d,1,H-4), 8.61 (s,1,H-7), 9.93-9.97 (d,1,H-11).

EXAMPLE 12

11-Amino-2-[2'-(dimethylamino)ethyl]-1,2-dihydro-3H-dibenz(deh)isoquinoline-1,3-dione (3)

A solution of 100 mg of 2-[2'-(dimethylamino)ethyl]-11-nitro-1,2-dihydro-3H-dibenz(deh)isoquinoline-1,3-dione in 100 ml of absolute ethanol was treated with 12 mg of palladium-on-carbon and shaken with hydrogen at 42 p.s.i. for 5 hours. The mixture was filtered and the filtrate was concentrated to give 91 mg (99%) of the title compound as a brown solid having a melting point of 150-152°C (melting point of dihydrochloride salt was above 300°C) and providng

the followng analysis:

$^1$H NMR ($d_6$DMSO, TS), δ values in ppm.

2.94 (s,6,N-$CH_3$), 3.30-3.60 (broad,2,$NH_2$), 3.63-3.70 (t,2,N-$CH_2$), 4.50-4.54 (t,2,CON-$CH_2$), 7.81-7.87 (t,1,H-9), 7.93-8.02 (q<<d over t>>,2,H-5 + H-10), 8.37-8.41 (d,1,H-8), 8.68-8.75 (t<<d over d>>,2,H-4 + H-6), 9.41 (s,1,H-7).

Example 13

Preparation of 7-Chloroanthracene-1,9-Dicarboxylic Acid Anhydride

A suspension of 2.0 g of 7-chloro-1,9-oxalylanthracene [Liebermann and Butescu, Chem. Ber., 45, 1213 (1912)] in 40 ml of p-dioxan was treated with 15 ml of 2N NaOH solution and 12 ml of 30% hydrogen peroxide. The ensuing exothermic reaction was controlled by cooling in an ice-water bath. After 40 minutes standing at room temperature, the resulting solution was acidified with dilute $H_2SO_4$ and the yellow precipitate that formed was collected by filtration, washed with water, and dried in air to give 2.14 g (95%) of the dicarboxylic acid. After recrystallization from p-dioxan and dimethylsulfoxide (4:1), it melted at 325-327°C (anhydride formed on heating).

A suspension of 2.0 g of the dicarboxylic acid in 50 ml of acetic anhydride was heated under reflux for 48 hours and then cooled to room temperature. The resulting orange solid, after being washed with ethanol and dried, afforded 1.84 g (97%) of the title compound. Recrystallization from p-dioxan and dimethylsulfoxide (4:1) gave orange crystals with melting point 325-327°C.

Example 14

10-Chloro-2-[2'-(dimethylamino)ethyl]-1,2-dihydro-3H-dibenz(deh)isoquinoline-1,3-dione (16)

A mixture of 1 g (3.6 mmol) of 7-chloroanthracene-1,9-dicarboxylic acid anhydride and 0.36 g (4 mmol) of N,N-dimethylethylenediamine in 70 ml of dry toluene was heated under reflux for 8 hours. The resulting solution was evaporated under reduced pressure and the orange residue was purified by column chromatography on silica gel with toluene-methanol (9:1) as solvent. This procedure gave 1.23 g (99%) of the title compound, crystallized from toluene, m.p. 165-167°C and providing the following analysis.

$^1$H NMR ($d_6$DMSO,TS), δ values in ppm.

2.4(s,6 ,N-$CH_3$), 2.56-2.80 (t,2,N-$CH_2$), 4.3-4.46(t,2,CON-$CH_2$), 7.45-7.60 (t,1,H-5), 7.68-7.78 (d,1,H-9), 7.87-7.97 (d,1,H-8), 8.19-8.29 (d,1,H-4), 8.62 (s over d,1,H-7),

8.62-8.72 (d over s,1,H-6), 9.93 (s,1,H-11).

Example 15

Preparation of 10-Choroanthracene-1,9-Dicarboxylic Acid Anhydride

To a cold (0°C) stirred mixture of 5.0 g (23.5 mmol) of 9-Chloroanthracene and 6.5 ml of oxalyl chloride in 35 ml of carbon disulfide was added 4 g of anhydrous aluminum chloride. After two hours additional carbon disulfide (15 ml) and aluminum chloride (4 g) were added. The mixture was stirred 2 more hours at 0°C and then overnight at room temperature. Dilute HCl was added and the orange precipitate that formed was collected by filtration, washed with water, and then digested well with 100 ml of 5% NaOH solution. The insoluble solids were collected, washed with water and dried in air to give 4.16 g (66%) of 10-chloro-1, 9-oxalyl-anthracene, m.p. 255-258°C, after crystallation from methanol containing a little p-dioxan. Acidification of the filtrate gave 1.83 g of 10-chloro-9-anthroic acid.

A suspension of 2 g (7.5 mmol) of 10-chloro-1,9-oxalylanthracene in 14 ml of 2N NaOH and 120 ml of p-dioxan at 15°C was treated portionwise with 14 ml of 30% hydrogen peroxide solution with shaking. After this addition was complete, the mixture was stirred at room temperature for 1 hour, and then diluted with 100 ml of water. Acidification with dilute $H_2SO_4$ resulted in the precipitation of 1.95 g (86%), after drying in air, of 10-chloroanthracene-1,9-dicarboxylic acid. It had a melting point 269-271°C (anhydride formed on heating) after crystallization from p-dioxan.

A suspension of 1.45 g (4.8 mmol) of 10-chloranthracene-1,9-dicarboxylic acid in 50 ml of acetic anhydride was heated under reflux for 4 hours and then cooled to room temperature. The yellow precipitate that formed was collected by filtration, washed with cold methanol and dried to give 0.83 g (61%) of the title compound, m.p. 269-271°C.

Example 16

7-Chloro-2-[2'-(dimethylamino)ethyl]-1,2-dihydro-3H-dibenz(deh)-isoquinoline-1,3-dione (17)

A mixture of 0.823 g (2.9 mmol) of 10-chloroanthracene-1,9-dicarboxylic acid anhydride and 0.256 g (3.0 mmol) of N,N-dimethylethylenediamine in 50 ml of dry toluene was heated under reflux for 48 hours. The resulting solution was evaporated to dryness and the residue was purified by column chromatography on silica gel with toluene-methanol (8:2) as solvent, affording a yellow solid that was purified further by preparative thin-layer chromatography on silica gel with toulene-methanol (9:1) as solvent. This procedure gave 0.71 g (69%) of the title compound, which had m.p. 169-171°C (decomposition) after recrystallization from hexane and toluene (4:1) and providing the following analysis.

$^1$H NMR ($CDCl_3$,TS), δ values in ppm.
2.4(s,6,N-$CH_3$), 2.65-2.78(t,2,14-$CH_2$), 4.3-4.46(t,2,CON-$CH_2$), 7.5-7.82 (m,3,H-5 + H-9 + H-10), 8.44-8.54 (d,1,H-8), 8.65-8.7 (d,2,H-4 + H-6), 9.88-9.98(d,1,H-11).

Example 17

2-[2'-(dimethylamino)ethyl]-1,2-dihydro-7-hydroxy-3H-dibenz(deh)isoquinoline-1,3-dione (21)

A solution of 50 mg (0.14 mmol) of 7-chloro-2-[2'-(dimethylamino)ethyl]-1,2-dihydro-3H-dibenz(deh)-isoquinoline-1,3-dione in 50 ml of methanol was treated with a solution of 12 mg (0.3 mmol) of NaOH in 1.5 ml of water. The mixture was stirred for 6 hours, treated with a few drops of glacial acetic acid, and evaporated to dryness. The residue was purified by preparative thin-layer chromatography on silica gel with toluene-methanol (9:1) as solvent to give 30 mg (63%) of the title compound, crystallized from hexane-toluene (1:1) containing a little methanol, as orange crystals with melting point 163-165°C and providing the following analysis.

$^1$H NMR ($CDCl_3$,TS), δ values in ppm.
2.4(s,6,N-$CH_3$), 2.70-2.73(t,2,N-$CH_2$), 4.23(s,1, OH), 4.40-4.43(t,2,CON-$CH_2$), 7.62-7.65 (t,1,H-5), 7.71-7.74(t, 1,H-9), 7.80-7.83(t,1,H-10), 8.40-8.44(d,1,H-8), 8.64-8.65 (d,1,H-4), 8.74-8.76 (d,1,H-6), 10.03-10.05 (d,1,H-10).
IR (KBr disk) 3440 $cm^{-1}$ (OH).

Example 18

2-[2'-(Dimethylamino)ethyl]-1,2-dihydro-7-methoxy-3H-dibenz-(deh)isoquinoline-1,3-dione (20)

A solution of 50 mg (0.14 mmol) of 7-chloro-2-[2'-(dimethylamino)ethyl]-1,2-dihydro-3H-dibenz(deh)-isoquinoline-1,3-dione in 25 ml of dry methanol was treated with a solution of 16.2 mg (0.30 mmol) of sodium methoxide in 2 ml of dry methanol. The mixture was stirred for 24 hours, treated with a few drops of glacial acetic acid, and concentrated under reduced pressure. The concentrate was purified by preparative thin-layer chromatography on silica gel with toluene-methanol (9:1) as solvent to give 43 mg (87%) of the title compound, crystallized from hexane-toluene (5:1) as red needles with melting point 147-149°C and providing the following analysis.

$^1$H NMR ($CDCl_3$,TS), δ values in ppm.
2.4(s,6,N-$CH_3$), 2.65-2.8(t,2,N-$CH_2$), 4.23(s,3, $OCH_3$), 4.33-4.48(t,2,CON-$CH_2$), 7.52-7.88 (m,3,H-5 + H-9 + H-10), 8.37-8.47(d,1,H-8), 8.58-8.78 (t<<d over d>>,2,H-4 + H-6), 9.95-10.06 (d,1,H-11).

Example 19

Preparation of 10-methylanthracene-1,9-Dicarboxylic Acid Anhydride

To a cold (0°C) stirred mixture of 5 g (26 mmol) of 9-methylanthracene and 6.5 ml of oxalyl chloride in 35 ml of carbon disulfide was added 4 g of anhydrous aluminum chloride. After two hours another 4 g of anhydrous aluminum chloride and 35 ml of carbon disulfide were added and stirring at 0°C was continued for two hours. The mixture was kept at room temperature overnight, treated with dilute HCl, and the orange precipitate that formed was collected by filtration, washed with water and then digested well with 100 ml of 5% NaOH solution. The insoluble solids were collected by filtration, washed with water and dried to give 3.17 g (50%) of 10-methyl-1,9-oxalylanthracene, m.p. 266-268°C, after crystallation from p-dioxan. Acidification of the filtrate with concentrated HCl gave 2.06 g of 10-methyl-9-anthroic acid.

A cold (10°C) suspension of 2.0 g (8.12 mmol) of 10-methyl-1,9-oxalylanthracene in 80 ml of p-dioxan and 15 ml of 2N NaOH was treated portionwise with 13 ml of 30% hydrogen peroxide with shaking. An exothermic reaction ensued and the solids dissolved gradually. After the addition was complete, the mixture was stirred for 40 minutes, and then acidified with dilute $H_2SO_4$. The resulting orange precipitate was collected by filtration, washed with water and dried to give 1.97 g (87%) of 10-methyl-1,9-anthracene dicarboxylic acid, which formed yellow crystals, m.p. 275-280°C (anhydride formed on heating) after recrystallization from chloroform-p-dioxan (2:1).

A suspension of 1.82 g (6.5 mmol) of the dicarboxylic acid in 25 ml of acetic anhydride was heated under reflux for 4 hours and then cooled to room temperature. The yellow solid was washed with ether and dried to afford 1.04 g of the title compound, m.p. 275-280°C. A further 0.27 g (total yield 77%) of this compound was obtained by evaporating the filtrate and digesting the residue twice with n-hexane.

Example 20

2-[2'-(Dimethylamino)ethyl]-1,2-dihydro-7-methyl-3H-dibenz(deh)isoquinoline-1,3-dione (19)

A mixture of 500 mg (1.91 mmol) of 10-methylanthracene-1,9-dicarboxylic acid anhydride and 2.0 mmol N,N-dimethylethylenediamine in 50 ml of dry toluene was heated under reflux for 5 hours. The solvent was evaporated and the residual solid was purified by column chromatography on silica gel with chloroform-methanol (9:1) as solvent. This procedure gave 605 mg (95%) of the title compound, crystallized from hexane-toluene (3:1), golden needles with m. p. 155-157°C and providing the following analysis.

$^1$H NMR ($CDCl_3$,TS), δ values in ppm
2.4(s,6,N-$CH_3$), 2.63-2.80(t,2,$NCH_2$), 3.08(s,3, $CH_3$), 4.29-4.46(t,2,CON-$CH_2$), 7.47-7.82 (m,3,H-5 + H-9 + H-10), 8.20-8.30(d,1,H-8), 8.48-8.58 (d,1,H-4), 8.59-8.69(d,1,H-6), 9.90-10.00(d,1,H-11).

Example 21

1,2-Dihydro-2-[2'-(methylamino)ethyl]-3H-dibenz(deh)isoquinoline-1,3-dione (22)

A mixture of 477 mg (1.5 mmol) of 2-[2'-(dimethylamino) ethyl]-1,2-dihydro-3H-dibenz(deh)isoquinoline-1,3-dione, 240 mg (1.5 mmol) of bromine, and 25 ml of glacial acetic acid was heated under reflux for 24 hours, cooled to room temperature, and diluted with ether. The solid that separated was dissolved in methanol and this solution was treated with methanolic KOH until it became slightly alkaline. It was then concentrated and the residue was separated into its components by column chromatography on silica gel with chloroform-methanol (19:1, then 9:1) as solvent. The first fraction (orange) gave 400 mg of unreacted starting material. The second fraction (green) gave a solid that was purified by preparative TLC on silica gel with chloroform-methanol (9:1) as solvent. This procedure gave 39 mg of the title compound, which formed a hydrochloride salt of m.p. 230-235°C (decomposition). The title compound provided the following analysis.

$^1$H NMR ($CDCl_3$,TS), δ values in ppm.
1.22(s,1,HN), 2.55(s,3,$NCH_3$), 3.06-3.11(t,2,$NCH_2$, 4.39-4.44(t,2,CON-$CH_2$), 7.52-7.66 (m,2,H-5 + H-9), 7.72-7.79 (t,1,H-10), 7.98-8.01(d,1,H-8), 8.21-8.24(d,1,H-4), 8.62-8.65(d,1,H-6), 8.66(s,1,H-7), 9,84-9.88(d,1,H-11).

Example 22

1,2-dihydro-2-[2'-(dimethylamino)ethyl]-1,2-dihydro-3H-dibenz(deh)-isoquinoline-1,3-dione-8-sulfonamide

The product of Example 1 is reacted with chlorosulfonic acid, followed by ammonia, and then dimethylethylenediamine in accordance with the procedure described in Example 2, to form the above-identified product.

Example 23

Preparation of a Mixture of 2-,6-, and 7-Acetamidoanthracene-1,9-dicarboxylic Acids

2-Acetamidoanthracene was prepared in 97% yield by stirring a solution of 1 equivalent of 2-aminoanthracene and 1.5 equivalents of acetic anhydride in dry tetrahydrofuran for 3 hours at room temperature. This product (2.9 g) was dissolved in 35 ml of carbon disulfide and 4 ml of oxalyl chloride was added. The stirred mixture was cooled to 0°C and treated with 2.5 g of anhydrous aluminum chloride. Another 35 ml of carbon disulfide and 2.5 g of aluminum

chloride were added after 2 hours. The mixture was stirred 2 hours at 0°C and overnight at room temperature and then treated with dilute HCl. The brown precipitate that formed was washed with water and then digested well with 5% NaOH solution. After collection, the insoluble solids were washed with water and dried in air to give 1.25 g (35%) of a mixture of 2-,6-, and 7-acetamido-1,9-oxalylanthracenes.

A suspension of the acetamidooxalylanthracenes (1.24 g, 4.27 mmol) in 25 ml of p-dioxane and 8 ml of 5% NaOH was treated at 15°C with 8 ml of 30% hydrogen peroxide. The mixture was stirred 45 minutes at room temperature, diluted with 50 ml of water, and filtered. The clear brown filtrate was acidified with dilute $H_2SO_4$ and the brick-red solid that formed was collected, washed well with water and dried in air to give 1.1 g (80%) of a mixture of the title compounds. This mixture was used directly in Example 24.

Example 24

4-, 9-, and 10-Acetamido-2-[2'-dimethylamino)ethyl]-1,2-dihydro-3H-dibenz(deh)isoquinoline-1,3-diones

A suspension of a mixture of 2-,. 6-, and 7-acetamido-1,9-dicarboxylic acids (1 g, 3.09 mmol) in 50 ml of dry toluene was heated under reflux with 310 mg (3.52 mmol) of N,N-dimethylethylenediamine for 15 hours. Anhydrous ethanol (10 ml) was added and reflux was continued another 5 hours. The mixture was concentrated under reduced pressure and the oily residue was chromatographed on silica gel with toluene-methanol as solvent. (8:2). Three fractions were obtained. The first fraction was purified further by preparative TLC on silica gel to give 27 mg (2%) of 4-acetamido-2-[2'-(dimethylamino)ethyl]-1,2-dihydro-3H-dibenz(deh)-isoquindine-1,3-dione as yellow solid. The second fraction gave 244 mg. of another isomeric acetamido derivative, melting point 249-252°C. An orange solid (714 mg) obtained from the third fraction was extracted with chloroform. The insoluble solid was washed with chlorofrom and dried in air to give 79 mg (7%) of the silicic acid salt of 10-acetamido-2-[2'-(dimethylamino)ethyl]-1,2-dihydro-3H-dibenz(deh)isoquinoline-1,3-dione, which did not melt below 360°C. Concentration of the chlorofom extract gave 632 mg (55%) of 9-acetamido-2-[2'-(dimethylamino)ethyl]-1,2-dihydro-3H-dibenz(deh)isoquinoline-1,3-dione, melting point 197-199°C after crystallization from toluene.

Example 25

9-Amino-2-[2'-(dimethylamino)ethyl]-1,2-dihydro-3H-dibenz(deh) isoquinoline-1,3-dione

A mixture of 210.3 mg of 9-acetamido-2-[2'-(dimethylamino)ethyl]-1,2-dihydro-3H-dibenz(deh)isoquinoline-1,3-diene, 50 ml of ethanol, and 5 ml of 37% HCl was heated under reflux for 2 hours and then concentrated to dryness. The residue was dissolved in methanol and this solution was made alkaline with methanolic KOH. It was then concentrated to a residue that was purified by preparative TLC on silica gel with toluene-methanol (9:1) as solvent. This procedure gave 162 mg (76%) of the title compound as a brick-red solid that had melting point 193-145°C after crystallization from toluene.

Example 26

10-Amino-2-[2'-(dimethylamino)ethyl]-1,2-dihydro-3H-dibenz(deh)isoquinoline-1,3-dione

The title compound was prepared by the procedure of Example 25. From 54 mg of 10-Acetamidomino-2-[2'-(dimethylamino)ethyl]-1,2-dihydro-3H-dibenz(deh)isoquinoline-1,3-diene was obtained 5 mg (10%) of the title compound.

Example 27

4-Amino-2-[2' (dimethylamino)ethyl]-1,2-dihydro-3H-dibenz(deh)isoquinoline-1,3-dione

The title compound was prepared by the procedure of Example 25. From 15 mg of 4-acetamido-2-[2'-(dimethylamino) ethyl]-1,2-dihydro-3H-dibenz(deh)isoquinoline-1,3-dione was obtained 7 mg (53%) of the title compound.

Example 28

2-[2'-(dimethylamino)ethyl]-1,2-dihydro-7-methylthio-3H-dibenz(deh)isoquinoline-1,3-dione

A solution of 50 mg (0.142 mmol) of 2-[2'-(dimethylamino) ethyl]-1,2-dihydro-7-chloro-3H-dibenz(deh)isoquinoline-1, 3-diene in 30 ml of warm anhydrous methanol was treated with 12 mg (0.17 mmol) of sodium thiomethoxide. The

mixture was stirred overnight at room temperature and concentrated to dryness. The residue was purified by preparative TLC on silica gel with toluene-methanol (9:1) as solvent to give 34 mg (66%) of the title compound, having a melting point of 137-139°C and providing the following analysis:

$^1H$ NMR ($CDCl_3$, TS), δ values in ppm.

2.35(s,6,N-$CH_3$), 2.40(s,3,S-$CH_3$), 2.58-2.75(t,2,N-$CH_2$), 4.27-4.42(t,2,$CONH_2$, 7.53-7.83(m,3,H-5 + H-9 +H-10), 8.65-8.73(d,1,H-8), 8.96-9.06(d,1,H-4), 9.18-9.28(d,1,H-6), 9.90-10.00(d,1,H-11).

Example 29

2-[2'-(Imidazolinyl)methyl]-1,2-dihydro-3H-dibenz(deh)isoquinolin-1,3-dione

By treating the compound prepared in Example 1 with amino acetonitrile followed by ethylene diamine dihydrochloride in accordance with the procedure described in Example 2, the above-identified compound can be prepared.

Example 30

Using Anthracene-1,9-Dicarboxylic Acid anhydride prepared in Example 1 and the appropriate amine, the following compounds can be prepared in accordance with the procedure described in Example 2:

2-[2'-(1-piperazinyl)ethyl]-1,2-dihydro-3H-dibenz(deh)isoquinoline-1,3-dione;
2-[2'-(N-morpholinyl)ethyl]-1,2-dihydro-3H-dibenz(deh)isoquinoline-1,3-dione;
2-[(1'-ethyl-2-pyrrolidinyl)methyl]-1,2-dihydro-3H-dibenz(deh)isoquinoline-1, 3-dione;
2-[2'-(1-methyl)-2-pyrrolidinyl)ethyl]-1,2-dihydro-3H-dibenz(deh)isoquinoline-1, 3-dione;
2-[(3'-piperidinyl)methyl]-1,2-dihydro-3H-dibenz(deh)isoquinoline-1,3-dione;
2-(3'-pyridyl)-1,2-dihydro-3H-dibenz(deh)isoquinoline-1,3-dione;
2-[2'-(2-pyridyl)ethyl]-1,2-dihydro-3H-dibenz(deh)isoquinoline-1,3-dione;
2-[(1'-aziridinyl)ethyl]-1,2-dihydro-3H-dibenz(deh)isoquinoline-1, 3-dione.

Example 31

Similarily, using the procedure described herein, the following derivatives of 2-(2'-(dimethylaminoethyl)-1,2-dihydro-3H-dibenz(deh)isoquinoline-1,3-dione can be prepared:

4-OH, $OCH_3$, $NO_2$, Cl, Br or $CF_3$;
5-OH, or $OCH_3$, Cl, or Br;
6-$NHCOCH_3$, $NH_2$, OH, $OCH_3$, Cl, Br, $CF_3$, $NO_2$, or $CH_3$;
7-$NHCOCH_3$, $NH_2$, Cl, Br or $CF_3$;
8-$NHCOCH_3$, OH, $OCH_3$, Cl, Br or $CF_3$;
9-OH, $OCH_3$, Cl, Br, $CF_3$ or $NO_2$;
10-OH, $OCH_3$, $CF_3$, Cl, Br or $NO_2$;
11-$NHCOCH_3$, Cl, OH, or $OCH_3$.

The $CF_3$ derivative is prepared from its corresponding bromo or chloro substituent by treatment with $CF_3CO_2Na$ and CuI, according to established techniques known in the art.

Example 32

2-[2'-(dimethylamino)ethyl]-1,2-dihydro-5-nitro-3H-dibenz(deh) isoqunioline-1,3-dione

A solution of 5-nitroindanone (Murray and Cromwell, J. Org. Chem. 41,3540 (1976)) in ethanol is treated with sodium borohydride followed by triphenylphosphine dibromide to furnish l-bromo-5-nitroindan, which is converted into a Grignard reagent by magnesium in ether. Addition of 2-cyclohexenone and cuprous iodide gives 1-(cyclohexanon-3-yl)-5-nitroindan. Conversion of this product into its hydroxymethylene derivative by ethyl formate and sodium ethoxide, followed by cyclization in the presence of polyphosphoric acid affords 7-nitro-4-oxo-1,2,3,4,8b, 11a-hexahydrocyclopentanoanthracene, which gives 3-nitro-1,9-cyclopentanoanthracene on reduction with sodium borohydride followed by treatment with dichlorodicyanobenzoquinone. Oxidation of this product with sodium dichromate, followed by

acidification, furnishes 5-nitroanthracene-1,9-dicarboxylic acid anhydride. Reaction of said anhydride with N,N-dimethylethylene diamine in accordance with the procedure described in Example 2 affords the above-identified product.

Example 33

5-Amino-2-[2'-(dimethylamino)ethyl]-1,2-dihydro-3H-dibenz(deh) isoquinoline-1,3-dione

The title compound is prepared by reducing the product prepared in Example 32 with hydrogen on palladium in accordance with the procedure described in Example 12.

Example 34

5-Acetamido-2-[2'-(dimethylamino)ethyl]-1,2-dihydro-3H-dibenz(deh)isoquinoline-1,3-dione

The title compound is prepared by treating the corresponding 5-amino derivative prepared in Example 33 with acetic anhydride in pyridine.

The compounds of the present invention were tested for anti-tumor activity in various model systems.

These models included the following:

1) In vitro tumor colony forming assays in soft agar with murine and human tumor cell lines and with fresh human tumors.
2) In vitro tumor cell viability assays using MTT dye reaction endpoints.
3) In vitro survival studies in mice bearing solid flank tumors or hematologic malignancies in the peritoneum.

For example, the compounds of the present invention were evaluated for cytotoxic activities in cloned human colon carcinomas. The clonogenic assays were conducted in accordance with the procedure described hereinbelow:

1) Colony Forming Assays in Soft Agar: Fresh human or murine tumors are disaggregated into single cell suspensions using mechanical, hypoosmotic and/or enzymatic (trypsin) methods. The single cells (about 5 X $10^4$-$10^5$) are plated in 35 mm plastic petri dishes onto a 1 ml "feeder layer" of 0.3% agar dissolved in growth medium containing 5-10% vol/vol of heat-inactivated fetal bovine serum, molten 0.3% agar and the antibiotics penicillin (100 u/mL) and streptomycin (100 ug/mL). Drug exposures can be performed for one hour or continuously (drugs added to final plating medium). Tumor cell colonies > 60 uM in size are counted by automated image analysis after 10-20 days of incubation in a humidified, 5-10% $CO_2$-gassed environment maintained at 37°C. Inhibition of colony formation is calculated based on comparisons to control (untreated) plates wherein the growth of hundreds of colonies/plate is typical. (Salmon SE, et al., N Engl J Med 298(24):1321-1327, 1978).

The results are indicated in the following tables.

Table 1.

| Activity of Compounds Against Tumor Cells | | | | |
|---|---|---|---|---|
| Concentration (u molar) for 50% inhibition of colony formation for human colon tumors | | | | |
| compd | LOVOp32 | 205p14 | SW80p105 | HT29p30 |
| 1 | 0.3 | 0.4 | 0.15 | 0.34 |
| 2 | 3.0 | 3.0 | 2.5 | 1.75 |
| 3 | 4.0 | 2.6 | 3.1 | 3.6 |
| 7 | 0.75 | 10.0 | 1.0 | 4.4 |
| 8 | 1.0 | 11.7 | 0.9 | 2.6 |
| 9 | 5.6 | 17.5 | 3.0 | 11.25 |
| 10 | NA | NA | 0.75 | 0.25 |
| 11 | 1.6 | 13.5 | 1.3 | 8.3 |
| 12 | 0.8 | 11.5 | 1.3 | 8.3 |
| Amonafide (control) | 0.6 | 0.16 | 0.57 | 0.96 |
| MTT assay with cells plated 24 hr. prior to drug addition. 3 day drug exposure. NA = not active at concentration tested | | | | |

Table 1a.

| Activities of Compounds Against Sensitive and Multidrug Resistant L1212 Leukemia Cells | | |
|---|---|---|
| Concentration (pg/ml) for 50% inhibition of tumor cells | | |
| Compound | Sensitive L1210 | Resistant L1210 |
| 1 | 0.0025 | 0.0025 |
| 13 | 0.0027 | 0.003 |
| 14 | 5.0 | ---- |
| 15 | 0.0031 | 0.0031 |
| 16 | 0.0025 | 0.002 |
| 17 | 0.028 | 0.03 |
| 19 | 0.003 | 0.003 |
| 20 | 0.0032 | 0.0028 |
| 21 | 0.0032 | 0.0032 |
| 22 | 0.0032 | 0.0027 |
| Six day MTT assay, continuous drug exposure | | |

Compounds of the present invention were tested for their in vitro activity in tumor cells sensitive and resistant to standard anti-cancer agents. The tumor cell lines used in this protocol are 8226 human Myeloma[1]; 8226/Dox-40[2], L-1210/Murine Leukemia, multidrug resistant L-210/[3], 2780 Human Ovarian Cancer and 2780/AD[4].

Each of these resistant cell lines is known as a multidrug resistant or "MDR" cell line. These cell lines produce a 170,00 molecular weight membrane protein termed the P-glycoprotein which acts as an active drug efflux pump. Thus, once a cell produces the P-glycoprotein, it has the capability of pumping out of the cell a large variety of unrelated natural products. These include some of our most active standard agents such as doxorubicin (adriamycin), vinca alkaloids (such as vincristine and vinblastine) and other DNA binders such as actinomycin D and daunomycin. The protocol for measuring the effectiveness of compounds of the present invention is as follows:

2) In Vitro Tumor Cell Viability Assays Using MTT-Dye: Tumors are processed into a single cell suspension as described above. The cells are plated at a concentration of 3-5 X $10^4$/1 mL well into plastic 96-well plates. Growth medium containing 5-10% (vol/vol) heat-inactivated fetal bovine serum and penicillin/streptomycin (as above) is added prior to incubation at 37°C for six days. Afterwards the medium containing the drug is removed, the cells are "washed" by centrifugation in fresh medium or phosphate-buffered saline (pH 7.4). A tetrazolium dye is then added (3,4,5-dimethylthiazol-2-yl)-2,5-diphenyl tetrazolium bromide (MTT). This dye forms a colored formazan product upon activation by mitochondrial reductases in viable cells.

Typically, the formazan product is solubilized in acid-propanol or DMSO. The intensity of the color is proportional to viable cell numbers and this is quantitated by spectrophotometric absorbance (570 nM) on a micro ELISA plate reader. Test results are calibrated in % control absorbance from untreated tumor cells (Mossman T: J Immunol Meth 65:55-63, 1983).

Using the above procedure, Compound 1 was tested for its cytotoxic activity with respect to various tumor cell lines. The results are indicated hereinbelow in Table 2.

TABLE 2

| In Vitro Cytotoxic Activity Insensitive and Multidrug Resistant Tumors* | | | |
|---|---|---|---|
| Tumor Cell Line | Resistance Spectrum | CMPD 1 Activity ($IC_{50}$ In ug/mL | CMPD 1 Cross Resistance |
| 8226 Human Myeloma | Multidrug resistant, P-glycoprotein prositive | .011 | 3-fold |

*4-day drug exposure in multiwell plastic plates; cell viability measured MTT dye reduction (N.C.I. method).

1. Matsuoka, Y, et al. Proc Soc Expt1. Biol. Med., 125, 1246-1250 (1967).
2. Dalton, W.S., et al. Cancer Research, 46, 5125-5130 (1986).
3. Dorr, et al., Biochem. Pharmacol., 36, 3115-3120 (1980).
4. Rogan, A.M., et al., Science, 224, 994-996 (1984).

TABLE 2 (continued)

| In Vitro Cytotoxic Activity Insensitive and Multidrug Resistant Tumors* | | | |
|---|---|---|---|
| Tumor Cell Line | Resistance Spectrum | CMPD 1 Activity ($IC_{50}$ In ug/mL | CMPD 1 Cross Resistance |
| 8226/DOX-40 | 40-fold resistant to Doxorubicin | .036 | |
| L-1210/Murine Leukemia | | .003 | |
| L-1210/ | MDR, P-glycoprotein (+) 10-fold resistance to Mitomycin C | .003 | None |
| 2780 Human Ovarian Cancer | | 4.0 | None |
| 2780/AD | MDR, P-glycoprotein (+) 10-fold resistance to Doxorubicin | 2.7 | |

*4-day drug exposure in multiwell plastic plates; cell viability measured MTT dye reduction (N.C.I. method).

Table 2 shows that Compound 1 maintained anti-tumor activity against these multidrug resistant tumors in vitro. The only instance in which Compound 1 did not appear to completely maintain its activity was with the DOX 40 cell line where a possible three-fold cross resistance was evident. however, this is a highly artificial level of resistance (i.e., 40-fold resistance is not seen commonly in the clinic). Thus, in the lower level resistant cell lines, such as the ten-fold mitomycin C resistant L1210 and the tenfold adriamycin resistant 2780 ovarian cancer, Compound 1 maintained its complete activity as shown in Table II.

The anti-tumor activity of compounds of the present invention in in vivo mouse murine models were studied.

3) Survival Studies In Tumor-Bearing Mice: 3.1 P-388 Leukemia Modles: One million P-388 leukemia cells originally obtained from American Type Culture Collection (Rockville, MD) are implanted into the peritoneum of adult DBA-2J male mice (Jackson Laboratories, Bar Harbor, ME). Twenty four hours later, drugs diluted in physiological saline are injected intraperitoneally at a volume of 0.1 mL/10 g body weight. The mice (10/group) are then followed for survival daily and compared to untreated tumor bearing mice. Survival results are converted to a percent increased lifespan over untreated controls (Geran RI, et al., Cancer Chemo Rep., 3, 1-10, 1972).

P-388/Adriamycin Resistant cells: The same protocol as above was used for these studies with a multidrug resistant P-388 cell line developed in vivo by and supplied by Dr. Randall Johnson (John RK, et al., Cancer Treat Rep 62, 1535-1547, 1978).

Colon-38: Freshly-harvested 20-30 mg pieces of viable colon-38 adenocarcinoma are injected into the right front flank of C57/B1 adult mice. These tumors are allowed to grow for three days. Drugs are injected intraperitoneally on days three and six after innoculation at a volume of 0.1 mL/10 g body weight. The perpendicular widths of the tumors are measured by caliper thrice weekly and converted to an estimated tumor mass according to the formula:

$$\frac{1 \text{ X } W^2}{2} = \text{grams of tumor.}$$

wherein

W = width of tumor

l = length of tumor

Tumor growth delay is calculated as the difference in days for tumors in treated mice to reach an estimated mass 750 mg or 1.5 g compared to that in untreated controls:

Days to reach 750 mg (Treated - Control) = Days of Tumor Growth Delay

Corbett, T.H., et al., Cancer Chemo Rep., 5 (1975). Mammary 16-C Adenocarcinoma and M5-76 Sarcoma: Chunks of tumor (20-50 mg) are subcutaneously implanted into the flank of B6C3F1 female mice. Drugs (10-45 mg/kg) are dissolved in saline and injected intraperitoneally every four days for three times starting one day after tumor implantation. Tumors are measured bidimensionally as described above and tumor growth delay is calculated at times to reach 1.5 and 3.0 g of tumor mass.

Using the various in vivo mouse models and the procedures described hereinabove, the anti-tumor activity of compound 1 was tested. The results are shown in Table 3 hereinbelow.

TABLE 3

ANTI-TUMOR ACTIVITY OF COMPOUND 1 in *in vivo* MOUSE MODELS

| mor Cell Line | Model | Drug Regimen (mg/kg X days) | Experimental Activity | Comparative Activity From The Literature: Amonafide | Doxorubicin | T-AMSA |
|---|---|---|---|---|---|---|
| P-388 lymphocytic leukemia | $10^6$ cells in DBA mice | 16; d1,5,9 | 00:ILS* | 99 ILS | 164 ILS | 124 ILS |
| P-388/ADR (adriamycin-Resistant) | $10^6$ cells in DBA mice | 15; d1,5,9 | 33 ILS | 35 ILS | 18 ILS | unk.** |
| Colon-38 Adenocarcinoma | 20mg implants in $C_{57}B$/mice | 15; d3,6 | 6 days TT | <7 days | 10 days | 2 days |
| 16-C Mammary Adenocarcinoma | (Southern Research Institute) | ---- | 7 days TT | 7 days | unk. | unk. |
| -76 Sarcoma | (Southern Research Institute) | ---- | 7 days | 7 days | unk. | unk. |

*ILS = Percentage Increased Lifespan
TT Days of Tumor Growth Delay
** Unknown

Drug deaths: a treated, tumored animal was presumed to die of drug toxicity if its day of death was significantly earlier than the corresponding day of death in the untreated control group and its tumor was less than 550 mg, or if it dies with a tumor of 550 mg or less prior to 45 days after the last day of treatment, or if the treated animal was uniquely specified as a drug death on data input. T-C excludes drug deaths, tumor free survivors and any other animal whose tumor failed to obtain the evaluation size and is the unweighted average of the differences of the median times post implant for the treated and control groups to attain each of the two evaluation sizes.

Evaluation of Tumor Growth

The appearance of tumors of a threshold size, and the growth in these tumors in groups of 10 mice (B6C3F0, 18-22 g) implanted with 5 X $10^6$ M5076 carcinoma cells compared to the tumor appearance and growth in samples treated with NSC308847 (amonafide) and Compound 1 were evaluated.

These studies confirm that the compound of this invention delays the appearance of tumors at the threshold level significantly over the control and similarly to the comparative drug (amonafide) at the same dose level, and significantly reduces the tumor growth at lower dose rates.

The cytotoxic activity of compounds of the present invention in fresh human tumors was also tested. The protocol is as follows:

Fresh human tumor specimens disaggregated to single cell suspensions and exposed to .001 ug/mL of drug continuously. Percent survival represents the fraction of tumor colonies (> 60 uM size) obtained after drug treatment compared to untreated cells of the same tumor. Overall sensitivity indicates tumor cell survival of less than 50% of control colony-forming cells. Each tumor sample is analyzed in three different petri dishes (i.e., n = 3 for each sample). The results are indicated in Table 4.

Table 4

CYTOTOXIC ACTIVITY FOR COMPOUND 1 IN FRESH HUMAN TUMORS[1]

| Human Tumor Type | Compound 1 Sensitive: No. of Samples | Compound 1 Sensitive: <30% Control | Compound 1 Sensitive: <30-50% of Control | Compound 1 Sensitive: Overall Response | Doxorubicin Sensitive[2]: No. of Samples | Doxorubicin Sensitive[2]: <30% Control | Doxorubicin Sensitive[2]: <30-50% of Control | Doxorubicin Sensitive[2]: Overall Response |
|---|---|---|---|---|---|---|---|---|
| Breast | 11 | 3 | 4 | 64% | 58 | 6 | 5 | 19% |
| Colon/Rectum | 7 | 8 | 1 | 14% | 17 | 3 | 2 | 29% |
| Lung | 6 | 2 | 1 | 50% | 12 | 1 | 1 | 17% |
| Melanoma | 11 | 4 | 3 | 64% | 10 | 0 | 0 | 0 |
| Ovary | 8 | 3 | 1 | 50% | 36 | 2 | 7 | 25% |
| Total | 43 | 12 | 10 | 51% | 133 | 12 | 15 | 20% |

[1]Fresh human tumor specimens disaggregated to single cell suspensions and exposed to .001 ug/mL of drug continuously. Percent survival represents the fraction of tumor colonies (> 60 uM size) obtained after drug treatment compared to untreated cells of the same tumor. Overall sensitivity indicates tumor cell survival of less than 50% of control colony-forming cells. Each tumor sample is analyzed in three different petri dishes (i.e., n = 3 for each sample).

[2]Doxorubicin tumors were from different patients.

The data in Table 4 results from testing on a group of human tumors, totalling 43 separate human cancers. The overall response rate in these 43 samples was 51% using a continuous drug concentration of (.001 ug/mL). These data represent a very high level of in vitro activity; some of the highest levels of activity were seen in typically adriamycin-responsive tumors such as breast cancer and ovarian cancer. Unexpected was the level of activity of compound 1 against melanomas (64%). Melanoma is widely known to be an extremely chemoresistant disease with most standard agents.[5] The data shows that compared to doxorubicin (overall response rate of 20%), compound 1 was significantly superior.

Other compounds were tested and the in vitro cytotoxic activity in accordance with the procedure described hereinabove with respect to Table 2. The results are indicated hereinbelow in Table 5.

TABLE 5

| IN VITRO CYTOTOXIC ACTIVITY | | | | |
|---|---|---|---|---|
| $IC_{50}$ VALUES (ng/ML CONTINUEOUS EXPOSURE)* | | | | |
| Compound # | 8226 Myeloma Sensitive | Cells Resistant | L1210 Leukemia Sensitive | Cells Resistant |
| 1 | 10 | 30 | 2.8 | 2.5 |
| 13 | 5 | 10 | 2.5 | 2.9 |
| 14 | >100 | >100 | 5,000 | >5,000 |

*Measured by MTT dye assay (N.C.I. Method).

The above preferred embodiments and examples are given to illustrate the scope and spirit of the present invention. These embodiments and examples will make apparent, to those skilled in the art, other embodiments and examples. These other embodiments and examples are within the contemplation of the present invention. Therefore, the present invention should be limited only by the appended claims.

## Claims

### Claims for the following Contracting States : AT, BE, CH, DE, DK, FR, GB, IT, LI, LU, NL, SE

1. A compound of the formula:

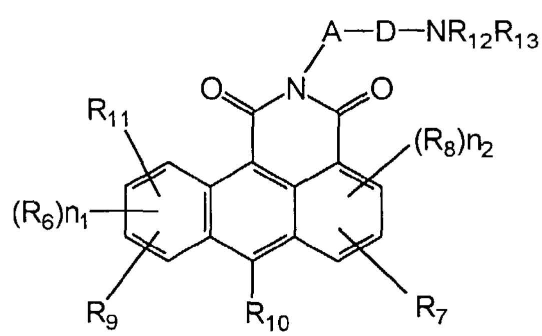

wherein

$R_8$, $R_6$ and $R_{10}$ are independently hydrogen, $C_1$-$C_6$ alkyl, aryl, $C_2$-$C_7$ alkanoyl, formyl, halogen, nitro, $NR_2R_3$, $OR_1$, or $SR_1$, hydroxy, methoxy, cyano, $CO_2H$, $SO_2NR_1R_2$, or $CONR_1R_2$;
$R_1$, $R_2$ and $R_3$ are independently hydrogen, $C_1$-$C_6$ alkyl, aryl-($C_1$-$C_6$-alkyl), aryl, formyl or $C_2$-$C_7$ alkanoyl; $R_9$, $R_{11}$, $R_{10}$ and $R_7$ are independently hydrogen, or $C_1$-$C_6$ alkyl or
$R_9$ and $R_{11}$ taken together with the carbon atoms to which they are attached form a phenyl group or
$R_9$ and $R_{10}$ taken together with the carbon atoms to which they are attached form a phenyl group or
$R_7$ and $R_{10}$ taken together with the carbon atoms to which they are attached form a phenyl group;

[5] Luce, J.K., Seminar Oncol. 2, 179-185 (1975).

A is $(CR_4R_5)n_3$, $C_3$-$C_{10}$ cycloalkyl or aryl or a chemical bond;
each $R_4$ and $R_5$ are independently hydrogen or $C_1$-$C_6$ alkyl;
$R_{12}$ and $R_{13}$ are independently hydrogen, or $C_1$-$C_6$ alkyl which is unsubstituted or substituted with hydroxy, mercapto, $C_1$-$C_6$ alkoxy, $C_2$-$C_7$ alkylcarbonyloxy, carboxy, or carbloweralkoxy or $R_{12}$ and $R_{13}$ taken together with the nitrogen to which they are attached form a 3-6-membered heterocyclic ring;
D is a chemical bond, or taken together with $NR_{12}$ form a 5 or 6-membered heterocyclic ring;
$n_1$ and $n_2$ are independently 0, 1 or 2; and
$n_3$ is 0, 1, 2, 3, 4 or 5.

**2.** The compound according to claim 1 wherein $R_9$, $R_{11}$, $R_{10}$, $R_7$ and $R_8$ are hydrogen.

**3.** The compound according to claim 1 or 2 wherein $R_6$ is hydrogen, amino, nitro, hydroxy or halo and n is 1.

**4.** The compound according to claim 3 wherein $R_6$ is 8-nitro, 8-amino, 11-nitro or 11-amino.

**5.** The compound according to any of claims 1 to 4 wherein A is aryl or $(CR_4R_5)n_3$ and D is a chemical bond.

**6.** The compound according to claim 5 wherein $n_3$ is 2-4.

**7.** The compound according to claim 6 wherein $R_4$ and $R_5$ are independently hydrogen.

**8.** The compound according to claim 1 wherein $R_{10}$ is hydrogen, methyl, chloro, methoxy or hydroxy.

**9.** The compound according to claim 1 wherein $R_{12}$ and $R_{13}$ are hydrogen or $C_1$-$C_6$ alkyl unsubstituted or substituted with hydroxy.

**10.** The compound according to claim 1 wherein $R_{12}$ and $R_{13}$ are the same.

**11.** The compound according to claim 1 wherein D taken together with $NR_{12}$ form a 5 or 6-membered nitrogen containing ring.

**12.** The compound according to claim 1 wherein $ADNR_{12}R_{13}$ is

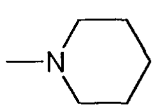 , 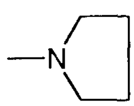 , 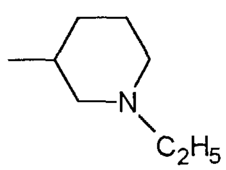 or 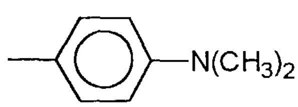

**13.** The compound according to claim 11 wherein $R_{12}$ and $R_{13}$ taken together with the nitrogen to which they are attached form a pyrrolidine or a piperidine ring.

**14.** The compound according to claim 1 which is

2-[2'-(N-pyrrolidino)ethyl]-1,2-dihydro-3H-dibenz(deh)isoquinoline-1,3-dione,
2-[2'-(N-piperidino)ethyl]-1,2-dihydro-3H-dibenz(deh)isoquinoline-1,3-dione,
2-(1'-ethyl-3'-piperidinyl)-1,2-dihydro-3H-dibenz(deh)isoquinoline-1,3-dione,
2-[3'-(bis-β-hydroxyethyl)aminopropyl]-1,2-dihydro-3H-dibenz(deh)isoquinoline-1,3-dione,
2-[3'-(dimethylamino)propyl]-1,2-dihydro-3H-dibenz(deh)isoquinoline-1,3-dione,
2-(4'-dimethylaminophenyl)-1,2-dihydro-3H-dibenz(deh)isoquinoline-1,3-dione,
2-[2'-(dimethylamino)ethyl]-1,2-dihydro-11-nitro-3H-dibenz(deh)isoquinoline-1,3-dione,
8-amino-2-[2'-(dimethylamino)ethyl]-1,2-dihydro-3H-dibenz(deh)isoquinoline-1,3-dione,
11-amino-2-[2'-(dimethylamino)ethyl]-1,2-dihydro-3H-dibenz(deh)isoquinoline-1,3-dione,
2-[2'-(dimethylamino)ethyl]-6-ethyl-1,2-dihydro-3H-dibenz(deh)isoquinoline-1,3-dione,
7-chloro-2-[2'-(dimethylamino)ethyl]-1,2-dihydro-3H-dibenz(deh)isoquinoline-1,3-dione,
2-[2'-(1-piperazinyl)ethyl]-1,2-dihydro-3H-dibenz(deh)isoquinoline-1,3-dione,

2-[2'-(N-morpholinyl)ethyl]-1,2-dihydro-3H-dibenz(deh)isoquinoline-1,3-dione,
2-[(1'-ethyl-2-pyrrolidinyl)methyl]-1,2-dihydro-3H-dibenz(deh)isoquinoline-1,3-dione,
2-[2'-(1-methyl)-2-pyrrolidinyl)ethyl]-1,2-dihydro-3H-dibenz(deh)isoquinoline-1,3-dione,
2-[(3'-piperidinyl)methyl]-1,2-dihydro-3H-dibenz(deh)isoquinoline-1,3-dione,
2-(3'-pyridyl)-1,2-dihydro-3H-dibenz(deh)isoquinoline-1,3-dione,
2-[2'-(2-pyridyl)ethyl]-1,2-dihydro-3H-dibenz(deh)isoquinoline-1,3-dione, and
2-[(1'-aziridinyl)ethyl]-1,2-dihydro-3H-dibenz(deh)isoquinoline-1,3-dione.

**15.** The compound according to claim 1 which is

2-[2'-(dimethylamino)ethyl]-1,2-dihydro-3H-dibenz(deh)isoquinoline-1,3-dione,
2-[2'-(dimethylamino)ethyl]-1,2-dihydro-8-nitro-3H-dibenz(deh)isoquinoline-1,3-dione,
2-[2'-(dimethylamino)ethyl]-1,2-dihydro-6-ethyl-3H-dibenz(deh)isoquinoline-1,3-dione,
10-chloro-2-[2'-(dimethylamino)ethyl]-1,2-dihydro-3H-dibenz(deh)isoquinoline-1,3-dione,
2-[2'-(dimethylamino)ethyl]-1,2-dihydro-7-hydroxy-3H-dibenz(deh)isoquinoline-1,3-dione,
2-[2'-(dimethylamino)ethyl]-1,2-dihydro-7-methoxy-3H-dibenz(deh)isoquinoline-1,3-dione,
2-[2'-(dimethylamino)ethyl]-1,2-dihydro-7-methyl-3H-dibenz(deh)isoquinoline-1,3-dione,
1,2-dihydro-2-[2'-methylaminoethyl]-3H-dibenz(deh)isoquinoline-1,3-dione,
4-acetamido-2-[(2'-dimethylamino)ethyl]-1,2-dihydro-3H-dibenz(deh)isoquinoline-1,3-dione,
4-amino-2-[(2'-dimethylamino)ethyl]-1,2-dihydro-3H-dibenz(deh)isoquinoline-1,3-dione,
4-hydroxy-2-[(2'-dimethylamino)ethyl]-1,2-dihydro-3H-dibenz(deh)isoquinoline-1,3-dione,
4-methoxy-2-[(2'-dimethylamino)ethyl]-1,2-dihydro-3H-dibenz(deh)isoquinoline-1,3-dione,
4-chloro-2-[(2'-dimethylamino)ethyl]-1,2-dihydro-3H-dibenz(deh)isoquinoline-1,3-dione,
4-trifluoromethyl-2-[(2'-dimethylamino)ethyl]-1,2-dihydro-3H-dibenz(deh)isoquinoline-1,3-dione,
5-acetamido-2-[(2'-dimethylamino)ethyl]-1,2-dihydro-3H-dibenz(deh)isoquinoline-1,3-dione,
5-amino-2-[(2'-dimethylamino)ethyl]-1,2-dihydro-3H-dibenz(deh)isoquinoline-1,3-dione,
5-methoxy-2-[(2'-dimethylamino)ethyl]-1,2-dihydro-3H-dibenz(deh)isoquinoline-1,3-dione, or
5-nitro-2-[(2'-dimethylamino)ethyl]-1,2-dihydro-3H-dibenz(deh)isoquinoline-1,3-dione.

**16.** The compound according to claim 1 which is

6-acetamido-2-[(2'-dimethylamino)ethyl]-1,2-dihydro-3H-dibenz(deh)isoquinoline-1,3-dione,
6-amino-2-[(2'-dimethylamino)ethyl]-1,2-dihydro-3H-dibenz(deh)isoquinoline-1,3-dione,
6-hydroxy-2-[(2'-dimethylamino)ethyl]-1,2-dihydro-3H-dibenz(deh)isoquinoline-1,3-dione,
6-methoxy-2-[(2'-dimethylamino)ethyl]-1,2-dihydro-3H-dibenz(deh)isoquinoline-1,3-dione,
6-chloro-2-[(2'-dimethylamino)ethyl]-1,2-dihydro-3H-dibenz(deh)isoquinoline-1,3-dione,
6-trifluoromethyl-2-[(2'-dimethylamino)ethyl]-1,2-dihydro-3H-dibenz(deh)isoquinoline-1,3-dione,
6-nitro-2-[(2'-dimethylamino)ethyl]-1,2-dihydro-3H-dibenz(deh)isoquinoline-1,3-dione,
6-methyl-2-[(2'-dimethylamino)ethyl]-1,2-dihydro-3H-dibenz(deh)isoquinoline-1,3-dione,
7-acetamido-2-[(2'-dimethylamino)ethyl]-1,2-dihydro-3H-dibenz(deh)isoquinoline-1,3-dione,
7-amino-2-[(2'-dimethylamino)ethyl]-1,2-dihydro-3H-dibenz(deh)isoquinoline-1,3-dione,
7-trifluoromethyl-2-[(2'-dimethylamino)ethyl]-1,2-dihydro-3H-dibenz(deh)isoquinoline-1,3-dione,
7-methylthio-2-[(2'-dimethylamino)ethyl]-1,2-dihydro-3H-dibenz(deh)isoquinoline-1,3-dione,
8-acetamido-2-[(2'-dimethylamino)ethyl]-1,2-dihydro-3H-dibenz(deh)isoquinoline-1,3-dione,
8-hydroxy-2-[(2'-dimethylamino)ethyl]-1,2-dihydro-3H-dibenz(deh)isoquinoline-1,3-dione,
8-methoxy-2-[(2'-dimethylamino)ethyl]-1,2-dihydro-3H-dibenz(deh)isoquinoline-1,3-dione,
8-chloro-2-[(2'-dimethylamino)ethyl]-1,2-dihydro-3H-dibenz(deh)isoquinoline-1,3-dione, or
8-trifluoromethyl-2-[(2'-dimethylamino)ethyl]-1,2-dihydro-3H-dibenz(deh)isoquinoline-1,3-dione.

**17.** The compound according to claim 1 which is

9-acetamido-2-[(2'-dimethylamino)ethyl]-1,2-dihydro-3H-dibenz(deh)isoquinoline-1,3-dione,
9-amino-2-[(2'-dimethylamino)ethyl]-1,2-dihydro-3H-dibenz(deh)isoquinoline-1,3-dione,
9-hydroxy-2-[(2'-dimethylamino)ethyl]-1,2-dihydro-3H-dibenz(deh)isoquinoline-1,3-dione,
9-methoxy-2-[(2'-dimethylamino)ethyl]-1,2-dihydro-3H-dibenz(deh)isoquinoline-1,3-dione,
9-chloro-2-[(2'-dimethylamino)ethyl]-1,2-dihydro-3H-dibenz(deh)isoquinoline-1,3-dione,
9-trifluoromethyl-2-[(2'-dimethylamino)ethyl]-1,2-dihydro-3H-dibenz(deh)isoquinoline-1,3-dione,
9-nitro-2-[(2'-dimethylamino)ethyl]-1,2-dihydro-3H-dibenz(deh)isoquinoline-1,3-dione,

10-acetamido-2-[(2'-dimethylamino)ethyl]-1,2-dihydro-3H-dibenz(deh)isoquinoline-1,3-dione,
10-amino-2-[(2'-dimethylamino)ethyl]-1,2-dihydro-3H-dibenz(deh)isoquinoline-1,3-dione,
10-hydroxy-2-[(2'-dimethylamino)ethyl]-1,2-dihydro-3H-dibenz(deh)isoquinoline-1,3-dione,
10-methoxy-2-[(2'-dimethylamino)ethyl]-1,2-dihydro-3H-dibenz(deh)isoquinoline-1,3-dione,
10-trifluoromethyl-2-[(2'-dimethylamino)ethyl]-1,2-dihydro-3H-dibenz(deh)isoquinoline-1,3-dione,
10-nitro-2-[(2'-dimethylamino)ethyl]-1,2-dihydro-3H-dibenz(deh)isoquinoline-1,3-dione,
11-acetamido-2-[(2'-dimethylamino)ethyl]-1,2-dihydro-3H-dibenz(deh)isoquinoline-1,3-dione,
11-hydroxy-2-[(2'-dimethylamino)ethyl]-1,2-dihydro-3H-dibenz(deh)isoquinoline-1,3-dione,
11-methoxy-2-[(2'-dimethylamino)ethyl]-1,2-dihydro-3H-dibenz(deh)isoquinoline-1,3-dione,
2-[2'-(dimethylamino)ethyl]-1,2-dihydro-5-nitro-3H-dibenz(deh)isoquinoline-1,3-dione,
5-amino-2-[(2'-dimethylamino)ethyl]-1,2-dihydro-3H-dibenz(deh)isoquinoline-1,3-dione, or
5-acetamido-2-[(2'-dimethylamino)ethyl]-1,2-dihydro-3H-dibenz(deh)isoquinoline-1,3-dione.

**18.** A pharmaceutical composition for the treatment of tumors comprising an anti-tumor effective amount of a compound according to any of claims 1 to 17 and a pharmaceutical carrier therefor.

**19.** A compound as defined in any of claims 1 to 17 for preparing a composition useful for treating tumors.

**Claims for the following Contracting States : ES, GR**

**1.** A process for preparing a compound of the formula I:

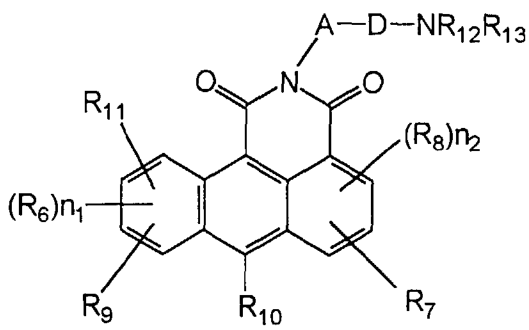

I

wherein

$R_8$, $R_6$ and $R_{10}$ are independently hydrogen, $C_1$-$C_6$ alkyl, aryl, $C_2$-$C_7$ alkanoyl, formyl, halogen, nitro, $NR_2R_3$, $OR_1$, or $SR_1$, hydroxy, methoxy, cyano, $CO_2H$, $SO_2NR_1R_2$, or $CONR_1R_2$;
$R_1$, $R_2$ and $R_3$ are independently hydrogen, $C_1$-$C_6$ alkyl, aryl-($C_1$-$C_6$-alkyl), aryl, formyl or $C_2$-$C_7$ alkanoyl; $R_9$, $R_{11}$, $R_{10}$ and $R_7$ are independently hydrogen, or $C_1$-$C_6$ alkyl or
$R_9$ and $R_{11}$ taken together with the carbon atoms to which they are attached form a phenyl group or
$R_9$ and $R_{10}$ taken together with the carbon atoms to which they are attached form a phenyl group or
$R_7$ and $R_{10}$ taken together with the carbon atoms to which they are attached form a phenyl group;
A is $(CR_4R_5)n_3$, $C_3$-$C_{10}$ cycloalkyl or aryl or a chemical bond;
each $R_4$ and $R_5$ are independently hydrogen or $C_1$-$C_6$ alkyl;
$R_{12}$ and $R_{13}$ are independently hydrogen, or $C_1$-$C_6$ alkyl which is unsubstituted or substituted with hydroxy, mercapto, $C_1$-$C_6$ alkoxy, $C_2$-$C_7$ alkylcarbonyloxy, carboxy, or carbloweralkoxy or $R_{12}$ and $R_{13}$ taken together with the nitrogen to which they are attached form a 3-6-membered heterocyclic ring;
D is a chemical bond, or taken together with $NR_{12}$ form a 5 or 6-membered heterocyclic ring;
$n_1$ and $n_2$ are independently 0, 1 or 2; and $n_3$ is 0, 1, 2, 3, 4 or 5,

wherein an anthracene-1,9-dicarboxylic anhydride of the formula II is condensed with an amine of the formula III:

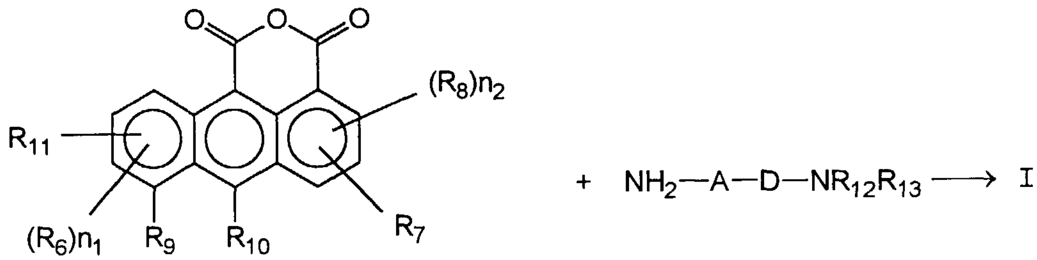

II III

wherein $R_1$, $R_2$, $R_3$, $R_4$, $R_5$, $R_6$, $R_7$, $R_8$, $R_9$, $R_{10}$, $R_{11}$, $R_{12}$, $R_{13}$, A, D, $n_1$, $n_2$ and $n_3$ are as defined above.

2. The process according to claim 1 wherein $R_7$, $R_8$, $R_9$, $R_{10}$ and $R_{11}$ are hydrogen.

3. The process according to claim 1 or 2 wherein $R_6$ is hydrogen, amino, nitro, hydroxy or halo and n is 1.

4. The process according to claim 3 wherein $R_6$ is 8-nitro, 8-amino, 11-nitro or 11-amino.

5. The process according to any of claims 1 to 4 wherein A is aryl or $(CR_4R_5)n_3$ and D is a chemical bond.

6. The process according to claim 5 wherein $n_3$ is 2-4.

7. The process according to claim 6 wherein $R_4$ and $R_5$ are independently hydrogen.

8. The process according to claim 1 wherein $R_{10}$ is hydrogen, methyl, chloro, methoxy or hydroxy.

9. The process according to claim 1 wherein $R_{12}$ and $R_{13}$ are hydrogen or $C_1$-$C_6$ alkyl unsubstituted or substituted with hydroxy.

10. The process according to claim 1 wherein $R_{12}$ and $R_{13}$ are the same.

11. The process according to claim 1 wherein D taken together with $NR_{12}$ form a 5 or 6-membered nitrogen containing ring.

12. The process according to claim 1 wherein $ADNR_{12}R_{13}$ is

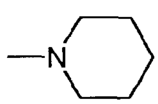 , 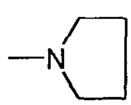 , 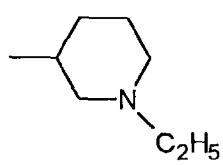 or 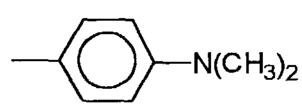

13. The process according to claim 11 wherein $R_{12}$ and $R_{13}$ taken together with the nitrogen to which they are attached form a pyrrolidine or a piperidine ring.

14. The process according to claim 1 wherein the prepared compound is selected from

2-[2'-(N-pyrrolidino)ethyl]-1,2-dihydro-3H-dibenz(deh)isoquinoline-1,3-dione,
2-[2'-(N-piperidino)ethyl]-1,2-dihydro-3H-dibenz(deh)isoquinoline-1,3-dione,
2-(1'-ethyl-3'-piperidinyl)-1,2-dihydro-3H-dibenz(deh)isoquinoline-1,3-dione,
2-[3'-(bis-β-hydroxyethyl)aminopropyl]-1,2-dihydro-3H-dibenz(deh)isoquinoline-1,3-dione,

2-[3'-(dimethylamino)propyl]-1,2-dihydro-3H-dibenz(deh)isoquinoline-1,3-dione,
2-(4'-dimethylaminophenyl)-1,2-dihydro-3H-dibenz(deh)isoquinoline-1,3-dione,
2-[2'-(dimethylamino)ethyl]-1,2-dihydro-11-nitro-3H-dibenz(deh)isoquinoline-1,3-dione,
8-amino-2-[2'-(dimethylamino)ethyl]-1,2-dihydro-3H-dibenz(deh)isoquinoline-1,3-dione,
11-amino-2-[2'-(dimethylamino)ethyl]-1,2-dihydro-3H-dibenz(deh)isoquinoline-1,3-dione,
2-[2'-(dimethylamino)ethyl]-6-ethyl-1,2-dihydro-3H-dibenz(deh)isoquinoline-1,3-dione,
7-chloro-2-[2'-(dimethylamino)ethyl]-1,2-dihydro-3H-dibenz(deh)isoquinoline-1,3-dione,
2-[2'-(1-piperazinyl)ethyl]-1,2-dihydro-3H-dibenz(deh)isoquinoline-1,3-dione,
2-[2'-(N-morpholinyl)ethyl]-1,2-dihydro-3H-dibenz(deh)isoquinoline-1,3-dione,
2-[(1'-ethyl-2-pyrrolidinyl)methyl]-1,2-dihydro-3H-dibenz(deh)isoquinoline-1,3-dione,
2-[2'-(1-methyl)-2-pyrrolidinyl)ethyl]-1,2-dihydro-3H-dibenz(deh)isoquinoline-1,3-dione,
2-[(3'-piperidinyl)methyl]-1,2-dihydro-3H-dibenz(deh)isoquinoline-1,3-dione,
2-(3'-pyridyl)-1,2-dihydro-3H-dibenz(deh)isoquinoline-1,3-dione,
2-[2'-(2-pyridyl)ethyl]-1,2-dihydro-3H-dibenz(deh)isoquinoline-1,3-dione, and
2-[(1'-aziridinyl)ethyl]-1,2-dihydro-3H-dibenz(deh)isoquinoline-1,3-dione.

**15.** The process according to claim 1 wherein the prepared compound is selected from

2-[2'-(dimethylamino)ethyl]-1,2-dihydro-3H-dibenz(deh)isoquinoline-1,3-dione,
2-[2'-(dimethylamino)ethyl]-1,2-dihydro-8-nitro-3H-dibenz(deh)isoquinoline-1,3-dione,
2-[2'-(dimethylamino)ethyl]-1,2-dihydro-6-ethyl-3H-dibenz(deh)isoquinoline-1,3-dione,
10-chloro-2-[2'-(dimethylamino)ethyl]-1,2-dihydro-3H-dibenz(deh)isoquinoline-1,3-dione,
2-[2'-(dimethylamino)ethyl]-1,2-dihydro-7-hydroxy-3H-dibenz(deh)isoquinoline-1,3-dione,
2-[2'-(dimethylamino)ethyl]-1,2-dihydro-7-methoxy-3H-dibenz(deh)isoquinoline-1,3-dione,
2-[2'-(dimethylamino)ethyl]-1,2-dihydro-7-methyl-3H-dibenz(deh)isoquinoline-1,3-dione,
1,2-dihydro-2-[2'-methylaminoethyl]-3H-dibenz(deh)isoquinoline-1,3-dione,
4-acetamido-2-[(2'-dimethylamino)ethyl]-1,2-dihydro-3H-dibenz(deh)isoquinoline-1,3-dione,
4-amino-2-[(2'-dimethylamino)ethyl]-1,2-dihydro-3H-dibenz(deh)isoquinoline-1,3-dione,
4-hydroxy-2-[(2'-dimethylamino)ethyl]-1,2-dihydro-3H-dibenz(deh)isoquinoline-1,3-dione,
4-methoxy-2-[(2'-dimethylamino)ethyl]-1,2-dihydro-3H-dibenz(deh)isoquinoline-1,3-dione,
4-chloro-2-[(2'-dimethylamino)ethyl]-1,2-dihydro-3H-dibenz(deh)isoquinoline-1,3-dione,
4-trifluoromethyl-2-[(2'-dimethylamino)ethyl]-1,2-dihydro-3H-dibenz(deh)isoquinoline-1,3-dione,
5-acetamido-2-[(2'-dimethylamino)ethyl]-1,2-dihydro-3H-dibenz(deh)isoquinoline-1,3-dione,
5-amino-2-[(2'-dimethylamino)ethyl]-1,2-dihydro-3H-dibenz(deh)isoquinoline-1,3-dione,
5-methoxy-2-[(2'-dimethylamino)ethyl]-1,2-dihydro-3H-dibenz(deh)isoquinoline-1,3-dione, or
5-nitro-2-[(2'-dimethylamino)ethyl]-1,2-dihydro-3H-dibenz(deh)isoquinoline-1,3-dione.

**16.** The process according to claim 1 wherein the prepared compound is selected from

6-acetamido-2-[(2'-dimethylamino)ethyl]-1,2-dihydro-3H-dibenz(deh)isoquinoline-1,3-dione,
6-amino-2-[(2'-dimethylamino)ethyl]-1,2-dihydro-3H-dibenz(deh)isoquinoline-1,3-dione,
6-hydroxy-2-[(2'-dimethylamino)ethyl]-1,2-dihydro-3H-dibenz(deh)isoquinoline-1,3-dione,
6-methoxy-2-[(2'-dimethylamino)ethyl]-1,2-dihydro-3H-dibenz(deh)isoquinoline-1,3-dione,
6-chloro-2-[(2'-dimethylamino)ethyl]-1,2-dihydro-3H-dibenz(deh)isoquinoline-1,3-dione,
6-trifluoromethyl-2-[(2'-dimethylamino)ethyl]-1,2-dihydro-3H-dibenz(deh)isoquinoline-1,3-dione,
6-nitro-2-[(2'-dimethylamino)ethyl]-1,2-dihydro-3H-dibenz(deh)isoquinoline-1,3-dione,
6-methyl-2-[(2'-dimethylamino)ethyl]-1,2-dihydro-3H-dibenz(deh)isoquinoline-1,3-dione,
7-acetamido-2-[(2'-dimethylamino)ethyl]-1,2-dihydro-3H-dibenz(deh)isoquinoline-1,3-dione,
7-amino-2-[(2'-dimethylamino)ethyl]-1,2-dihydro-3H-dibenz(deh)isoquinoline-1,3-dione,
7-trifluoromethyl-2-[(2'-dimethylamino)ethyl]-1,2-dihydro-3H-dibenz(deh)isoquinoline-1,3-dione,
7-methylthio-2-[(2'-dimethylamino)ethyl]-1,2-dihydro-3H-dibenz(deh)isoquinoline-1,3-dione,
8-acetamido-2-[(2'-dimethylamino)ethyl]-1,2-dihydro-3H-dibenz(deh)isoquinoline-1,3-dione,
8-hydroxy-2-[(2'-dimethylamino)ethyl]-1,2-dihydro-3H-dibenz(deh)isoquinoline-1,3-dione,
8-methoxy-2-[(2'-dimethylamino)ethyl]-1,2-dihydro-3H-dibenz(deh)isoquinoline-1,3-dione,
8-chloro-2-[(2'-dimethylamino)ethyl]-1,2-dihydro-3H-dibenz(deh)isoquinoline-1,3-dione, or
8-trifluoromethyl-2-[(2'-dimethylamino)ethyl]-1,2-dihydro-3H-dibenz(deh)isoquinoline-1,3-dione.

**17.** The process according to claim 1 wherein the prepared compound is selected from

9-acetamido-2-[(2'-dimethylamino)ethyl]-1,2-dihydro-3H-dibenz(deh)isoquinoline-1,3-dione,
9-amino-2-[(2'-dimethylamino)ethyl]-1,2-dihydro-3H-dibenz(deh)isoquinoline-1,3-dione,
9-hydroxy-2-[(2'-dimethylamino)ethyl]-1,2-dihydro-3H-dibenz(deh)isoquinoline-1,3-dione,
9-methoxy-2-[(2'-dimethylamino)ethyl]-1,2-dihydro-3H-dibenz(deh)isoquinoline-1,3-dione,
9-chloro-2-[(2'-dimethylamino)ethyl]-1,2-dihydro-3H-dibenz(deh)isoquinoline-1,3-dione,
9-trifluoromethyl-2-[(2'-dimethylamino)ethyl]-1,2-dihydro-3H-dibenz(deh)isoquinoline-1,3-dione,
9-nitro-2-[(2'-dimethylamino)ethyl]-1,2-dihydro-3H-dibenz(deh)isoquinoline-1,3-dione,
10-acetamido-2-[(2'-dimethylamino)ethyl]-1,2-dihydro-3H-dibenz(deh)isoquinoline-1,3-dione,
10-amino-2-[(2'-dimethylamino)ethyl]-1,2-dihydro-3H-dibenz(deh)isoquinoline-1,3-dione,
10-hydroxy-2-[(2'-dimethylamino)ethyl]-1,2-dihydro-3H-dibenz(deh)isoquinoline-1,3-dione,
10-methoxy-2-[(2'-dimethylamino)ethyl]-1,2-dihydro-3H-dibenz(deh)isoquinoline-1,3-dione,
10-trifluoromethyl-2-[(2'-dimethylamino)ethyl]-1,2-dihydro-3H-dibenz(deh)isoquinoline-1,3-dione,
10-nitro-2-[(2'-dimethylamino)ethyl]-1,2-dihydro-3H-dibenz(deh)isoquinoline-1,3-dione,
11-acetamido-2-[(2'-dimethylamino)ethyl]-1,2-dihydro-3H-dibenz(deh)isoquinoline-1,3-dione,
11-hydroxy-2-[(2'-dimethylamino)ethyl]-1,2-dihydro-3H-dibenz(deh)isoquinoline-1,3-dione,
11-methoxy-2-[(2'-dimethylamino)ethyl]-1,2-dihydro-3H-dibenz(deh)isoquinoline-1,3-dione,
2-[2'-(dimethylamino)ethyl]-1,2-dihydro-5-nitro-3H-dibenz(deh)isoquinoline-1,3-dione,
5-amino-2-[(2'-dimethylamino)ethyl]-1,2-dihydro-3H-dibenz(deh)isoquinoline-1,3-dione, or
5-acetamido-2-[(2'-dimethylamino)ethyl]-1,2-dihydro-3H-dibenz(deh)isoquinoline-1,3-dione.

**18.** A process for preparing a pharmaceutical composition comprising the step of mixing an anti-tumor effective amount of a compound as prepared according to any of claims 1 to 17 with a pharmaceutical carrier therefor.

**19.** The use of a compound as prepared in any of claims 1 to 17 for preparing a medicament useful for treating tumors.

## Patentansprüche

## Patentansprüche für folgende Vertragsstaaten : AT, BE, CH, DE, DK, FR, GB, IT, LI, LU, NL, SE

**1.** Verbindung der Formel:

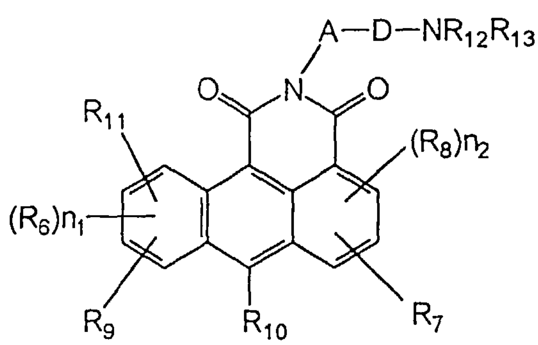

worin

$R_8$, $R_6$ und $R_{10}$ unabhängig Wasserstoff, $C_1$-$C_6$-Alkyl, Aryl, $C_2$-$C_7$-Alkanoyl, Formyl, Halogen, Nitro, $NR_2R_3$, $OR_1$ oder $SR_1$, Hydroxy, Methoxy, Cyano, $CO_2H$, $SO_2NR_1R_2$ oder $CONR_1R_2$ sind;
$R_1$, $R_2$ und $R_3$ unabhängig Wasserstoff, $C_1$-$C_6$-Alkyl, Aryl-($C_1$-$C_6$-alkyl), Aryl, Formyl oder $C_2$-$C_7$-Alkanoyl sind;
$R_9$, $R_{11}$, $R_{10}$ und $R_7$ unabhängig Wasserstoff oder $C_1$-$C_6$-Alkyl sind oder
$R_9$ und $R_{11}$, zusammen mit den Kohlenstoffatomen, an die sie gebunden sind, eine Phenyl-Gruppe bilden oder
$R_9$ und $R_{10}$, zusammen mit den Kohlenstoffatomen, an die sie gebunden sind, eine Phenyl-Gruppe bilden oder
$R_7$ und $R_{10}$, zusammen mit den Kohlenstoffatomen, an die sie gebunden sind, eine Phenyl-Gruppe bilden;
A $(CR_4R_5)n_3$, $C_3$-$C_{10}$-Cycloalkyl oder Aryl oder eine chemische Bindung ist;
$R_4$ und $R_5$ jeweils unabhängig Wasserstoff oder $C_1$-$C_6$-Alkyl sind;

$R_{12}$ und $R_{13}$ sind unabhängig Wasserstoff oder $C_1$-$C_6$-Alkyl, das unsubstituiert oder substituiert mit Hydroxy, Mercapto, $C_1$-$C_6$-Alkoxy, $C_2$-$C_7$-Alkylcarbonyloxy, Carboxy oder Carbo-niederalkoxy ist, oder $R_{12}$ und $R_{13}$ bilden zusammen mit dem Stickstoffatom, an das sie gebunden sind, einen 3- bis 6-gliedrigen heterocyclischen Ring;
D ist eine chemische Bindung oder bildet zusammen mit $NR_{12}$ einen 5- oder 6-gliedrigen heterocyclischen Ring;
$n_1$ und $n_2$ sind unabhängig 0, 1 oder 2; und
$n_3$ ist 0, 1, 2, 3, 4 oder 5.

**2.** Verbindung gemäß Anspruch 1, worin $R_9$, $R_{11}$, $R_{10}$, $R_7$ und $R_8$ Wasserstoff sind.

**3.** Verbindung gemäß Anspruch 1 oder 2, worin $R_6$ Wasserstoff, Amino, Nitro, Hydroxy oder Halo ist und n 1 ist.

**4.** Verbindung gemäß Anspruch 3, worin $R_6$ 8-Nitro, 8-Amino, 11-Nitro oder 11-Amino ist.

**5.** Verbindung gemäß einem der Ansprüche 1 bis 4, worin A Aryl oder $(CR_4R_5)n_3$ ist und D eine chemische Bindung ist.

**6.** Verbindung gemäß Anspruch 5, worin $n_3$ 2 bis 4 ist.

**7.** Verbindung gemäß Anspruch 6, worin $R_4$ und $R_5$ unabhängig Wasserstoff sind.

**8.** Verbindung gemäß Anspruch 1, worin $R_{10}$ Wasserstoff, Methyl, Chlor, Methoxy oder Hydroxy ist.

**9.** Verbindung gemäß Anspruch 1, worin $R_{12}$ und $R_{13}$ Wasserstoff oder $C_1$-$C_6$-Alkyl, unsubstituiert oder substituiert mit Hydroxy, sind.

**10.** Verbindung gemäß Anspruch 1, worin $R_{12}$ und $R_{13}$ gleich sind.

**11.** Verbindung gemäß Anspruch 1, worin D zusammen mit $NR_{12}$ einen 5-oder 6-gliedrigen stickstoffhaltigen Ring bildet.

**12.** Verbindung gemäß Anspruch 1, worin $ADNR_{12}R_{13}$

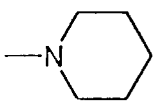 , 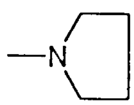 , 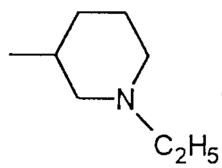 oder 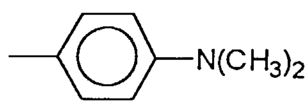

ist.

**13.** Verbindung gemäß Anspruch 11, worin $R_{12}$ und $R_{13}$ zusammen mit dem Stickstoffatom an das sie gebunden sind, einen Pyrrolidin- oder Piperidin-Ring bilden.

**14.** Verbindung gemäß Anspruch 1, welche

2-[2'-(N-Pyrrolidino)ethyl]-1,2-dihydro-3H-dibenz(deh)isochinolin-1,3-dion,
2-[2'-(N-Piperidino)ethyl]-1,2-dihydro-3H-dibenz(deh)isochinolin-1,3-dion,
2-(1'-Ethyl-3'-piperidinyl)-1,2-dihydro-3H-dibenz(deh)isochinolin-1,3-dion,
2-[3'-(Bis-β-hydroxyethyl)aminopropyl]-1,2-dihydro-3H-dibenz(deh)isochinolin-1,3-dion,
2-[3'-(Dimethylamino)propyl]-1,2-dihydro-3H-dibenz(deh)isochinolin-1,3-dion,
2-(4'-Dimethylaminophenyl)-1,2-dihydro-3H-dibenz(deh)isochinolin-1,3-dion,
2-[2'-(Dimethylamino)ethyl]-1,2-dihydro-11-nitro-3H-dibenz(deh)isochinolin-1,3-dion,
8-Amino-2-[2'-(dimethylamino)ethyl]-1,2-dihydro-3H-dibenz(deh)isochinolin-1,3-dion,
11-Amino-2-[2'-(dimethylamino)ethyl]-1,2-dihydro-3H-dibenz(deh)isochinolin-1,3-dion,
2-[2'-(Dimethylamino)ethyl]-6-ethyl-1,2-dihydro-3H-dibenz(deh)isochinolin-1,3-dion,

7-Chloro-2-[2'-(dimethylamino)ethyl]-1,2-dihydro-3H-dibenz(deh)isochinolin-1,3-dion,
2-[2'-(1-Piperazinyl)ethyl]-1,2-dihydro-3H-dibenz(deh)isochinolin-1,3-dion,
2-[2'-(N-Morpholinyl)ethyl]-1,2-dihydro-3H-dibenz(deh)isochinolin-1,3-dion,
2-[(1'-Ethyl-2-pyrrolidinyl)methyl]-1,2-dihydro-3H-dibenz(deh)isochinolin-1,3-dion,
2-[2'-(1-Methyl)-2-pyrrolidinyl)ethyl]-1,2-dihydro-3H-dibenz(deh)isochinolin-1,3-dion,
2-[(3'-Piperidinyl)methyl]-1,2-dihydro-3H-dibenz(deh)isochinolin-1,3-dion,
2-(3'-Pyridyl)-1,2-dihydro-3H-dibenz(deh)isochinolin-1,3-dion,
2-[2'-(2-Pyridyl)ethyl]-1,2-dihydro-3H-dibenz(deh)isochinolin-1,3-dion und
2-[(1'-Aziridinyl)ethyl]-1,2-dihydro-3H-dibenz(deh)isochinolin-1,3-dion

ist.

**15.** Verbindung gemäß Anspruch 1, welche

2-[2'-(Dimethylamino)ethyl]-1,2-dihydro-3H-dibenz(deh)isochinolin-1,3-dion,
2-[2'-(Dimethylamino)ethyl]-1,2-dihydro-8-nitro-3H-dibenz(deh)isochinolin-1,3-dion,
2-[2'-(Dimethylamino)ethyl]-1,2-dihydro-6-ethyl-3H-dibenz(deh)isochinolin-1,3-dion,
10-Chlor-2-[2'-(dimethylamino)ethyl]-1,2-dihydro-3H-dibenz(deh)isochinolin-1,3-dion,
2-[2'-(Dimethylamino)ethyl]-1,2-dihydro-7-hydroxy-3H-dibenz(deh)isochinolin-1,3-dion,
2-[2'-(Dimethylamino)ethyl]-1,2-dihydro-7-methoxy-3H-dibenz(deh)isochinolin-1,3-dion,
2-[2'-(Dimethylamino)ethyl]-1,2-dihydro-7-methyl-3H-dibenz(deh)isochinolin-1,3-dion,
1,2-Dihydro-2-[2'-methylaminoethyl]-3H-dibenz(deh)isochinolin-1,3-dion,
4-Acetamido-2-[(2'-dimethylamino)ethyl]-1,2-dihydro-3H-dibenz(deh)isochinolin-1,3-dion,
4-Amino-2-[(2'-dimethylamino)ethyl]-1,2-dihydro-3H-dibenz(deh)isochinolin-1,3-dion,
4-Hydroxy-2-[(2'-dimethylamino)ethyl]-1,2-dihydro-3H-dibenz(deh)isochinolin-1,3-dion,
4-Methoxy-2-[(2'-dimethylamino)ethyl]-1,2-dihydro-3H-dibenz(deh)isochinolin-1,3-dion,
4-Chloro-2-[(2'-dimethylamino)ethyl]-1,2-dihydro-3H-dibenz(deh)isochinolin-1,3-dion,
4-Trifluormethyl-2-[(2'-dimethylamino)ethyl]-1,2-dihydro-3H-dibenz(deh)isochinolin-1,3-dion,
5-Acetamido-2-[(2'-dimethylamino)ethyl]-1,2-dihydro-3H-dibenz(deh)isochinolin-1,3-dion,
5-Amino-2-[(2'-dimethylamino)ethyl]-1,2-dihydro-3H-dibenz(deh)isochinolin-1,3-dion,
5-Methoxy-2-[(2'-dimethylamino)ethyl]-1,2-dihydro-3H-dibenz(deh)isochinolin-1,3-dion oder
5-Nitro-2-[(2'-dimethylamino)ethyl]-1,2-dihydro-3H-dibenz(deh)isochinolin-1,3-dion ist.

**16.** Verbindung gemäß Anspruch 1, welche

6-Acetamido-2-[(2'-dimethylamino)ethyl]-1,2-dihydro-3H-dibenz(deh)isochinolin-1,3-dion,
6-Amino-2-[(2'-dimethylamino)ethyl]-1,2-dihydro-3H-dibenz(deh)isochinolin-1,3-dion,
6-Hydroxy-2-[(2'-dimethylamino)ethyl]-1,2-dihydro-3H-dibenz(deh)isochinolin-1,3-dion,
6-Methoxy-2-[(2'-dimethylamino)ethyl]-1,2-dihydro-3H-dibenz(deh)isochinolin-1,3-dion,
6-Chlor-2-[(2'-dimethylamino)ethyl]-1,2-dihydro-3H-dibenz(deh)isochinolin-1,3-dion,
6-Trifluormethyl-2-[(2'-dimethylamino)ethyl]-1,2-dihydro-3H-dibenz(deh)isochinolin-1,3-dion,
6-Nitro-2-[(2'-dimethylamino)ethyl]-1,2-dihydro-3H-dibenz(deh)isochinolin-1,3-dion,
6-Methyl-2-[(2'-dimethylamino)ethyl]-1,2-dihydro-3H-dibenz(deh)isochinolin-1,3-dion,
7-Acetamido-2-[(2'-dimethylamino)ethyl]-1,2-dihydro-3H-dibenz(deh)isochinolin-1,3-dion,
7-Amino-2-[(2'-dimethylamino)ethyl]-1,2-dihydro-3H-dibenz(deh)isochinolin-1,3-dion,
7-Trifluormethyl-2-[(2'-dimethylamino)ethyl]-1,2-dihydro-3H-dibenz(deh)isochinolin-1,3-dion,
7-Methylthio-2-[(2'-dimethylamino)ethyl]-1,2-dihydro-3H-dibenz(deh)isochinolin-1,3-dion,
8-Acetamido-2-[(2'-dimethylamino)ethyl]-1,2-dihydro-3H-dibenz(deh)isochinolin-1,3-dion,
8-Hydroxy-2-[(2'-dimethylamino)ethyl]-1,2-dihydro-3H-dibenz(deh)isochinolin-1,3-dion,
8-Methoxy-2-[(2'-dimethylamino)ethyl]-1,2-dihydro-3H-dibenz(deh)isochinolin-1,3-dion,
8-Chlor-2-[(2'-dimethylamino)ethyl]-1,2-dihydro-3H-dibenz(deh)isochinolin-1,3-dion oder
8-Trifluormethyl-2-[(2'-dimethylamino)ethyl]-1,2-dihydro-3H-dibenz(deh)isochinolin-1,3-dion ist.

**17.** Verbindung gemäß Anspruch 1, welche

9-Acetamido-2-[(2'-dimethylamino)ethyl]-1,2-dihydro-3H-dibenz(deh)isochinolin-1,3-dion,
9-Amino-2-[(2'-dimethylamino)ethyl]-1,2-dihydro-3H-dibenz(deh)isochinolin-1,3-dion,
9-Hydroxy-2-[(2'-dimethylamino)ethyl]-1,2-dihydro-3H-dibenz(deh)isochinolin-1,3-dion,

9-Methoxy-2-[(2'-dimethylamino)ethyl]-1,2-dihydro-3H-dibenz(deh)isochinolin-1,3-dion,
9-Chlor-2-[(2'-dimethylamino)ethyl]-1,2-dihydro-3H-dibenz(deh)isochinolin-1,3-dion,
9-Trifluormethyl-2-[(2'-dimethylamino)ethyl]-1,2-dihydro-3H-dibenz(deh)isochinolin-1,3-dion,
9-Nitro-2-[(2'-dimethylamino)ethyl]-1,2-dihydro-3H-dibenz(deh)isochinolin-1,3-dion,
10-Acetamido-2-[(2'-dimethylamino)ethyl]-1,2-dihydro-3H-dibenz(deh)isochinolin-1,3-dion,
10-Amino-2-[(2'-dimethylamino)ethyl]-1,2-dihydro-3H-dibenz(deh)isochinolin-1,3-dion,
10-Hydroxy-2-[(2'-dimethylamino)ethyl]-1,2-dihydro-3H-dibenz(deh)isochinolin-1,3-dion,
10-Methoxy-2-[(2'-dimethylamino)ethyl]-1,2-dihydro-3H-dibenz(deh)isochinolin-1,3-dion,
10-Trifluormethyl-2-[(2'-dimethylamino)ethyl]-1,2-dihydro-3H-dibenz(deh)isochinolin-1,3-dion,
10-Nitro-2-[(2'-dimethylamino)ethyl]-1,2-dihydro-3H-dibenz(deh)isochinolin-1,3-dion,
11-Acetamido-2-[(2'-dimethylamino)ethyl]-1,2-dihydro-3H-dibenz(deh)isochinolin-1,3-dion,
11-Hydroxy-2-[(2'-dimethylamino)ethyl]-1,2-dihydro-3H-dibenz(deh)isochinolin-1,3-dion,
11-Methoxy-2-[(2'-dimethylamino)ethyl]-1,2-dihydro-3H-dibenz(deh)isochinolin-1,3-dion,
2-[2'-(Dimethylamino)ethyl]-1,2-dihydro-5-nitro-3H-dibenz(deh)isochinolin-1,3-dion,
5-Amino-2-[(2'-dimethylamino)ethyl]-1,2-dihydro-3H-dibenz(deh)isochinolin-1,3-dion oder
5-Acetamido-2-[(2'-dimethylamino)ethyl]-1,2-dihydro-3H-dibenz(deh)isochinolin-1,3-dion ist.

**18.** Pharmazeutische Zusammensetzung zur Behandlung von Tumoren, umfassend eine gegen Tumoren wirksame Menge einer Verbindung gemäß einem der Ansprüche 1 bis 17 und einen pharmazeutischen Träger dafür.

**19.** Verbindung gemäß einem der Ansprüche 1 bis 17 zur Herstellung einer Zusammensetzung, die zur Behandlung von Tumoren verwendbar ist.

**Patentansprüche für folgende Vertragsstaaten : ES, GR**

**1.** Verfahren zur Herstellung einer Verbindung der Formel I:

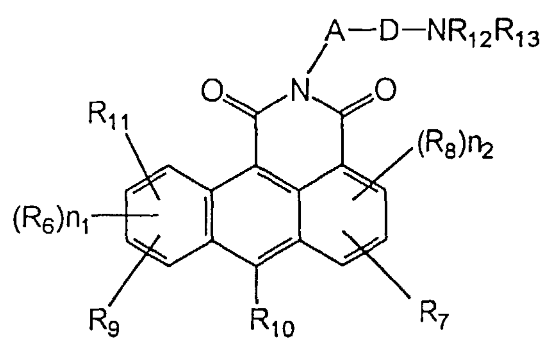

I

worin

$R_8$, $R_6$ und $R_{10}$ unabhängig Wasserstoff, $C_1$-$C_6$-Alkyl, Aryl, $C_2$-$C_7$-Alkanoyl, Formyl, Halogen, Nitro, $NR_2R_3$, $OR_1$ oder $SR_1$, Hydroxy, Methoxy, Cyano, $CO_2H$, $SO_2NR_1R_2$ oder $CONR_1R_2$ sind;
$R_1$, $R_2$ und $R_3$ unabhängig Wasserstoff, $C_1$-$C_6$-Alkyl, Aryl-($C_1$-$C_6$-alkyl), Aryl, Formyl oder $C_2$-$C_7$-Alkanoyl sind;
$R_9$, $R_{11}$, $R_{10}$ und $R_7$ unabhängig Wasserstoff oder $C_1$-$C_6$- Alkyl sind oder
$R_9$ und $R_{11}$, zusammen mit den Kohlenstoffatomen, an die sie gebunden sind, eine Phenyl-Gruppe bilden oder
$R_9$ und $R_{10}$, zusammen mit den Kohlenstoffatomen, an die sie gebunden sind, eine Phenyl-Gruppe bilden oder
$R_7$ und $R_{10}$, zusammen mit den Kohlenstoffatomen, an die sie gebunden sind, eine Phenyl-Gruppe bilden;
A $(CR_4R_5)n_3$, $C_3$-$C_{10}$-Cycloalkyl oder Aryl oder eine chemische Bindung ist;
$R_4$ und $R_5$ jeweils unabhängig Wasserstoff oder $C_1$-$C_6$-Alkyl sind;
$R_{12}$ und $R_{13}$ sind unabhängig Wasserstoff oder $C_1$-$C_6$-Alkyl, das unsubstituiert oder substituiert mit Hydroxy, Mercapto, $C_1$-$C_6$-Alkoxy, $C_2$-$C_7$-Alkylcarbonyloxy, Carboxy oder Carbo-niederalkoxy ist, oder $R_{12}$ und $R_{13}$ bilden zusammen mit dem Stickstoffatom, an das sie gebunden sind, einen 3- bis 6-gliedrigen heterocyclischen Ring;
D ist eine chemische Bindung oder bildet zusammen mit $NR_{12}$ einen 5- oder 6-gliedrigen heterocyclischen

Ring;
$n_1$ und $n_2$ sind unabhängig 0, 1 oder 2; und
$n_3$ ist 0, 1, 2, 3, 4 oder 5,

worin ein Anthracen-1,9-dicarbonsäureanhydrid der Formel II mit einem Amin der Formel III kondensiert wird:

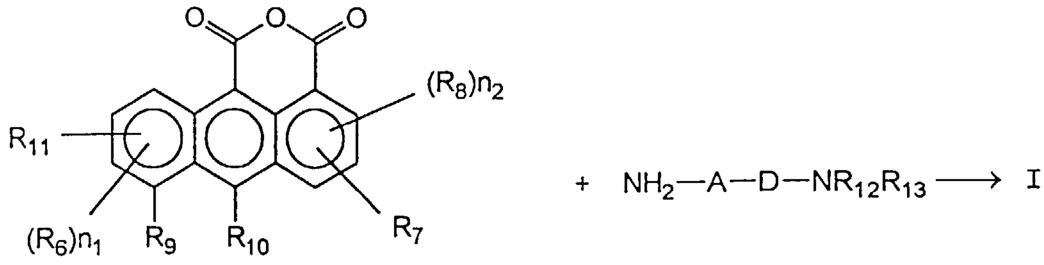

II III

worin $R_1$, $R_2$, $R_3$, $R_4$, $R_5$, $R_6$, $R_7$, $R_8$, $R_9$, $R_{10}$, $R_{11}$, $R_{12}$, $R_{13}$, A, D, $n_1$, $n_2$ und $n_3$ wie oben definiert sind.

**2.** Verfahren gemäß Anspruch 1, worin $R_7$, $R_8$, $R_9$, $R_{10}$ und $R_{11}$ Wasserstoff sind.

**3.** Verfahren gemäß Anspruch 1 oder 2, worin $R_6$ Wasserstoff, Amino, Nitro, Hydroxy oder Halo ist und n 1 ist.

**4.** Verfahren gemäß Anspruch 3, worin $R_6$ 8-Nitro, 8-Amino, 11-Nitro oder 11-Amino ist.

**5.** Verfahren gemäß einem der Ansprüche 1 bis 4, worin A Aryl oder $(CR_4R_5)n_3$ ist und D eine chemische Bindung ist.

**6.** Verfahren gemäß Anspruch 5, worin $n_3$ 2 bis 4 ist.

**7.** Verfahren gemäß Anspruch 6, worin $R_4$ und $R_5$ unabhängig Wasserstoff sind.

**8.** Verfahren gemäß Anspruch 1, worin $R_{10}$ Wasserstoff, Methyl, Chlor, Methoxy oder Hydroxy ist.

**9.** Verfahren gemäß Anspruch 1, worin $R_{12}$ und $R_{13}$ Wasserstoff oder $C_1$-$C_6$-Alkyl, unsubstituiert oder substituiert mit Hydroxy, sind.

**10.** Verfahren gemäß Anspruch 1, worin $R_{12}$ und $R_{13}$ gleich sind.

**11.** Verfahren gemäß Anspruch 1, worin D zusammen mit $NR_{12}$ einen 5-oder 6-gliedrigen Stickstoff-haltigen Ring bildet.

**12.** Verfahren gemäß Anspruch, worin $ADNR_{12}R_{13}$

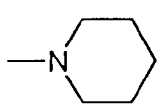 , 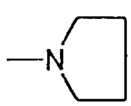 , 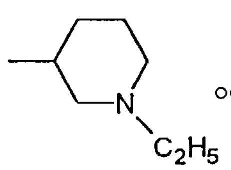 oder 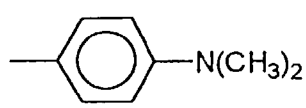

ist.

**13.** Verfahren gemäß Anspruch 11, worin $R_{12}$ und $R_{13}$ zusammen mit dem Stickstoffatom, an das sie gebunden sind, einen Pyrrolidin- oder Piperidin-Ring bilden.

**14.** Verfahren gemäß Anspruch 1, wobei die hergestellte Verbindung ausgewählt wird aus:

2-[2'-(N-Pyrrolidino)ethyl]-1,2-dihydro-3H-dibenz(deh)isochinolin-1,3-dion,
2-[2'-(N-Piperidino)ethyl]-1,2-dihydro-3H-dibenz(deh)isochinolin-1,3-dion,
2-(1'-Ethyl-3'-piperidinyl)-1,2-dihydro-3H-dibenz(deh)isochinolin-1,3-dion,
2-[3'-(Bis-β-hydroxyethyl)aminopropyl]-1,2-dihydro-3H-dibenz(deh)isochinolin-1,3-dion,
2-[3'-(Dimethylamino)propyl]-1,2-dihydro-3H-dibenz(deh)isochinolin-1,3-dion,
2-(4'-Dimethylaminophenyl)-1,2-dihydro-3H-dibenz(deh)isochinolin-1,3-dion,
2-[2'-(Dimethylamino)ethyl]-1,2-dihydro-11-nitro-3H-dibenz(deh)isochinolin-1,3-dion,
8-Amino-2-[2'-(dimethylamino)ethyl]-1,2-dihydro-3H-dibenz(deh)isochinolin-1,3-dion,
11-Amino-2-[2'-(dimethylamino)ethyl]-1,2-dihydro-3H-dibenz(deh)isochinolin-1,3-dion,
2-[2'-(Dimethylamino)ethyl]-6-ethyl-1,2-dihydro-3H-dibenz(deh)isochinolin-1,3-dion,
7-Chloro-2-[2'-(dimethylamino)ethyl]-1,2-dihydro-3H-dibenz(deh)isochinolin-1,3-dion,
2-[2'-(1-Piperazinyl)ethyl]-1,2-dihydro-3H-dibenz(deh)isochinolin-1,3-dion,
2-[2'-(N-Morpholinyl)ethyl]-1,2-dihydro-3H-dibenz(deh)isochinolin-1,3-dion,
2-[(1'-Ethyl-2-pyrrolidinyl)methyl]-1,2-dihydro-3H-dibenz(deh)isochinolin-1,3-dion,
2-[2'-(1-Methyl)-2-pyrrolidinyl)ethyl]-1,2-dihydro-3H-dibenz(deh)isochinolin-1,3-dion,
2-[(3'-Piperidinyl)methyl]-1,2-dihydro-3H-dibenz(deh)isochinolin-1,3-dion,
2-(3'-Pyridyl)-1,2-dihydro-3H-dibenz(deh)isochinolin-1,3-dion,
2-[2'-(2-Pyridyl)ethyl] -1,2-dihydro-3H-dibenz(deh)isochinolin-1,3-dion und
2-[(1'-Aziridinyl)ethyl]-1,2-dihydro-3H-dibenz(deh)isochinolin-1,3-dion.

**15.** Verfahren gemäß Anspruch 1, worin die hergestellte Verbindung ausgewählt wird aus:

2-[2'-(Dimethylamino)ethyl]-1,2-dihydro-3H-dibenz(deh)isochinolin-1,3-dion,
2-[2'-(Dimethylamino)ethyl]-1,2-dihydro-8-nitro-3H-dibenz(deh)isochinolin-1,3-dion,
2-[2'-(Dimethylamino)ethyl]-1,2-dihydro-6-ethyl-3H-dibenz(deh)isochinolin-1,3-dion,
10-Chlor-2-[2'-(dimethylamino)ethyl]-1,2-dihydro-3H-dibenz(deh)isochinolin-1,3-dion,
2-[2'-(Dimethylamino)ethyl]-1,2-dihydro-7-hydroxy-3H-dibenz(deh)isochinolin-1,3-dion,
2-[2'-(Dimethylamino)ethyl]-1,2-dihydro-7-methoxy-3H-dibenz(deh)isochinolin-1,3-dion,
2-[2'-(Dimethylamino)ethyl]-1,2-dihydro-7-methyl-3H-dibenz(deh)isochinolin-1,3-dion,
1,2-Dihydro-2-[2'-methylaminoethyl]-3H-dibenz(deh)isochinolin-1,3-dion,
4-Acetamido-2-[(2'-dimethylamino)ethyl]-1,2-dihydro-3H-dibenz(deh)isochinolin-1,3-dion,
4-Amino-2-[(2'-dimethylamino)ethyl]-1,2-dihydro-3H-dibenz(deh)isochinolin-1,3-dion,
4-Hydroxy-2-[(2'-dimethylamino)ethyl]-1,2-dihydro-3H-dibenz(deh)isochinolin-1,3-dion,
4-Methoxy-2-[(2'-dimethylamino)ethyl]-1,2-dihydro-3H-dibenz(deh)isochinolin-1,3-dion,
4-Chloro-2-[(2'-dimethylamino)ethyl]-1,2-dihydro-3H-dibenz(deh)isochinolin-1,3-dion,
4-Trifluormethyl-2-[(2'-dimethylamino)ethyl]-1,2-dihydro-3H-dibenz(deh)isochinolin-1,3-dion,
5-Acetamido-2-[(2'-dimethylamino)ethyl]-1,2-dihydro-3H-dibenz(deh)isochinolin-1,3-dion,
5-Amino-2-[(2'-dimethylamino)ethyl]-1,2-dihydro-3H-dibenz(deh)isochinolin-1,3-dion,
5-Methoxy-2-[(2'-dimethylamino)ethyl]-1,2-dihydro-3H-dibenz(deh)isochinolin-1,3-dion oder
5-Nitro-2-[(2'-dimethylamino)ethyl]-1,2-dihydro-3H-dibenz(deh)isochinolin-1,3-dion.

**16.** Verfahren gemäß Anspruch 1, worin die hergestellte Verbindung ausgewählt wird aus:

6-Acetamido-2-[(2'-dimethylamino)ethyl]-1,2-dihydro-3H-dibenz(deh)isochinolin-1,3-dion,
6-Amino-2-[(2'-dimethylamino)ethyl]-1,2-dihydro-3H-dibenz(deh)isochinolin-1,3-dion,
6-Hydroxy-2-[(2'-dimethylamino)ethyl]-1,2-dihydro-3H-dibenz(deh)isochinolin-1,3-dion,
6-Methoxy-2-[(2'-dimethylamino)ethyl]-1,2-dihydro-3H-dibenz(deh)isochinolin-1,3-dion,
6-Chlor-2-[(2'-dimethylamino)ethyl]-1,2-dihydro-3H-dibenz(deh)isochinolin-1,3-dion,
6-Trifluormethyl-2-[(2'-dimethylamino)ethyl]-1,2-dihydro-3H-dibenz(deh)isochinolin-1,3-dion,
6-Nitro-2-[(2 '-dimethylamino)ethyl]-1,2-dihydro-3H-dibenz(deh)isochinolin-1,3-dion,
6-Methyl-2-[(2'-dimethylamino)ethyl]-1,2-dihydro-3H-dibenz(deh)isochinolin-1,3-dion,
7-Acetamido-2-[(2'-dimethylamino)ethyl]-1,2-dihydro-3H-dibenz(deh)isochinolin-1,3-dion,
7-Amino-2-[(2'-dimethylamino)ethyl]-1,2-dihydro-3H-dibenz(deh)isochinolin-1,3-dion,
7-Trifluormethyl-2-[(2'-dimethylamino)ethyl]-1,2-dihydro-3H-dibenz(deh)isochinolin-1,3-dion,
7-Methylthio-2-[(2'-dimethylamino)ethyl]-1,2-dihydro-3H-dibenz(deh)isochinolin-1,3-dion,
8-Acetamido-2-[(2'-dimethylamino)ethyl]-1,2-dihydro-3H-dibenz(deh)isochinolin-1,3-dion,

8-Hydroxy-2-[(2'-dimethylamino)ethyl]-1,2-dihydro-3H-dibenz(deh)isochinolin-1,3-dion,
8-Methoxy-2-[(2'-dimethylamino)ethyl]-1,2-dihydro-3H-dibenz(deh)isochinolin-1,3-dion,
8-Chlor-2-[(2'-dimethylamino)ethyl]-1,2-dihydro-3H-dibenz(deh)isochinolin-1,3-dion oder
8-Trifluormethyl-2-[(2'-dimethylamino)ethyl]-1,2-dihydro-3H-dibenz(deh)isochinolin-1,3-dion.

**17.** Verfahren gemäß Anspruch 1, worin die hergestellte Verbindung ausgewählt wird aus:

9-Acetamido-2-[(2'-dimethylamino)ethyl]-1,2-dihydro-3H-dibenz(deh)isochinolin-1,3-dion,
9-Amino-2-[(2'-dimethylamino)ethyl]-1,2-dihydro-3H-dibenz(deh)isochinolin-1,3-dion,
9-Hydroxy-2-[(2'-dimethylamino)ethyl]-1,2-dihydro-3H-dibenz(deh)isochinolin-1,3-dion,
9-Methoxy-2-[(2'-dimethylamino)ethyl]-1,2-dihydro-3H-dibenz(deh)isochinolin-1,3-dion,
9-Chlor-2-[(2'-dimethylamino)ethyl]-1,2-dihydro-3H-dibenz(deh)isochinolin-1,3-dion,
9-Trifluormethyl-2-[(2'-dimethylamino)ethyl]-1,2-dihydro-3H-dibenz(deh)isochinolin-1,3-dion,
9-Nitro-2-[(2'-dimethylamino)ethyl]-1,2-dihydro-3H-dibenz(deh)isochinolin-1,3-dion,
10-Acetamido-2-[(2'-dimethylamino)ethyl]-1,2-dihydro-3H-dibenz(deh)isochinolin-1,3-dion,
10-Amino-2-[(2'-dimethylamino)ethyl]-1,2-dihydro-3H-dibenz(deh)isochinolin-1,3-dion,
10-Hydroxy-2-[(2'-dimethylamino)ethyl]-1,2-dihydro-3H-dibenz(deh)isochinolin-1,3-dion,
10-Methoxy-2-[(2'-dimethylamino)ethyl]-1,2-dihydro-3H-dibenz(deh)isochinolin-1,3-dion,
10-Trifluormethyl-2-[(2'-dimethylamino)ethyl]-1,2-dihydro-3H-dibenz(deh)isochinolin-1,3-dion,
10-Nitro-2-[(2'-dimethylamino)ethyl]-1,2-dihydro-3H-dibenz(deh)isochinolin-1,3-dion,
11-Acetamido-2-[(2'-dimethylamino)ethyl]-1,2-dihydro-3H-dibenz(deh)isochinolin-1,3-dion,
11-Hydroxy-2-[(2'-dimethylamino)ethyl]-1,2-dihydro-3H-dibenz(deh)isochinolin-1,3-dion,
11-Methoxy-2-[(2'-dimethylamino)ethyl]-1,2-dihydro-3H-dibenz(deh)isochinolin-1,3-dion,
2-[2'-(Dimethylamino)ethyl]-1,2-dihydro-5-nitro-3H-dibenz(deh)isochinolin-1,3-dion,
5-Amino-2-[(2'-dimethylamino)ethyl]-1,2-dihydro-3H-dibenz(deh)isochinolin-1,3-dion oder
5-Acetamido-2-[(2'-dimethylamino)ethyl]-1,2-dihydro-3H-dibenz(deh)isochinolin-1,3-dion.

**18.** Verfahren zur Herstellung einer pharmazeutischen Zusammensetzung, umfassend den Schritt der Mischung einer gegen Tumor wirksamen Menge einer Verbindung, wie hergestellt gemäß einem der Ansprüche 1 bis 17 mit einem pharmazeutischen Träger dafür.

**19.** Verwendung einer Verbindung wie hergestellt in einem der Ansprüche 1 bis 17, zur Herstellung eines Medikaments, das zur Behandlung von Tumoren verwendbar ist.

**Revendications**

**Revendications pour les Etats contractants suivants : AT, BE, CH, DE, DK, FR, GB, IT, LI, LU, NL, SE**

**1.** Composé de formule :

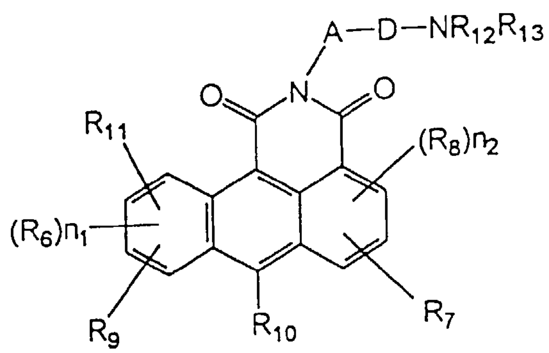

dans laquelle

$R_8$, $R_6$ et $R_{10}$ sont chacun indépendamment des autres un atome d'hydrogène, un groupe alkyle en $C_1$-$C_6$, aryle, alcanoyle en $C_2$-$C_7$, formyle, halogéno, nitro, $NR_2R_3$, $OR_1$, ou $SR_1$, hydroxy, méthoxy, cyano, $CO_2H$, $SO_2NR_1R_2$, ou $CONR_1R_2$ ;
$R_1$, $R_2$ et $R_3$ sont chacun indépendamment des autres un atome d'hydrogène, un groupe alkyle en $C_1$-$C_6$, aryl (alkyle en $C_1$-$C_6$), aryle, formyle ou alcanoyle en $C_2$-$C_7$ ;
$R_9$, $R_{11}$, $R_{10}$ et $R_7$ sont chacun indépendamment des autres un atome d'hydrogène ou un groupe alkyle en $C_1$-$C_6$ ; ou
$R_9$ et $R_{11}$, avec les atomes de carbone auxquels ils sont liés, forment un groupe phényle, ou
$R_9$ et $R_{10}$, avec les atomes de carbone auxquels ils sont liés, forment un groupe phényle, ou
$R_7$ et $R_{10}$, avec les atomes de carbone auxquels ils sont liés, forment un groupe phényle ;
A est $(CR_4R_5)n_3$, un groupe cycloalkyle en $C_3$-$C_{10}$ ou aryle ou une liaison chimique ;
$R_4$ et $R_5$ sont chacun indépendamment de l'autre un atome d'hydrogène ou un groupe alkyle en $C_1$-$C_6$ ;
$R_{12}$ et $R_{13}$ sont chacun indépendamment de l'autre un atome d'hydrogène ou un groupe alkyle en $C_1$-$C_6$ qui est non substitué ou substitué par des substituants hydroxy, mercapto, alcoxy en $C_1$-$C_6$, (alkyle en en $C_2$-$C_7$) carbonyloxy, carbonyle, ou carb(alcoxy inférieur), ou $R_{12}$ et $R_{13}$, avec l'atome d'azote auquel ils sont liés, forment un noyau hétérocyclique ayant de 3 à 6 chaînons ;
D est une liaison chimique, ou encore, avec $NR_{12}$, forme un noyau hétérocyclique à 5 ou 6 chaînons ;
$n_1$ et $n_2$ valent chacun indépendamment de l'autre 0, 1 ou 2 ; et
$n_3$ vaut 0, 1, 2, 3, 4 ou 5.

**2.** Composé selon la revendication 1, dans lequel $R_9$, $R_{11}$, $R_{10}$, $R_7$ et $R_8$ sont des atomes d'hydrogène.

**3.** Composé selon la revendication 1 ou 2, dans lequel $R_6$ est un atome d'hydrogène ou un groupe amino, nitro, hydroxy ou halogéno, et n vaut 1.

**4.** Composé selon la revendication 3, dans lequel $R_6$ est le groupe 8-nitro, 8-amino, 11-nitro ou 11-amino.

**5.** Composé selon l'une quelconque des revendications 1 à 4, dans lequel A est un groupe aryle ou $(CR_4R_5)n_3$ et D est une liaison chimique.

**6.** Composé selon la revendication 5, dans lequel $n_3$ vaut de 2 à 4.

**7.** Composé selon la revendication 6, dans lequel $R_4$ et $R_5$ sont chacun indépendamment de l'autre un atome d'hydrogène.

**8.** Composé selon la revendication 1, dans lequel $R_{10}$ est un atome d'hydrogène ou le groupe méthyle, chlore, méthoxy ou hydroxy.

**9.** Composé selon la revendication 1, dans lequel $R_{12}$ et $R_{13}$ sont des atomes d'hydrogène ou des groupes alkyle en $C_{1-6}$ non substitués ou substitués par des substituants hydroxy.

**10.** Composé selon la revendication 1, dans lequel $R_{12}$ et $R_{13}$ sont identiques.

**11.** Composé selon la revendication 1, dans lequel D, avec $NR_{12}$, forme un noyau azoté ayant 5 ou 6 chaînons.

**12.** Composé selon la revendication 1, dans lequel $ADNR_{12}R_{13}$ est

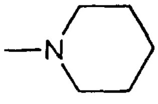 , 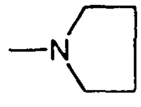 , 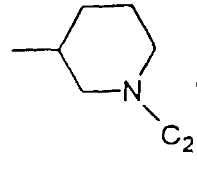 ou 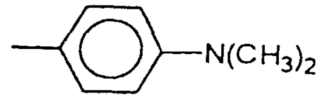

**13.** Composé selon la revendication 11, dans lequel $R_{12}$ et $R_{13}$, avec l'atome d'azote auquel ils sont liés, forment un noyau pyrrolidine ou pipéridine.

**14.** Composé selon la revendication 1, qui est l'un des composés suivants :

2-[2'-(N-pyrrolidino)éthyl]-1,2-dihydro-3H-dibenzo(deh)isoquinoléine-1,3-dione,
2-[2'-(N-pipéridino)éthyl]-1,2-dihydro-3H-dibenzo(deh)isoquinoléine-1,3-dione,
2-(1'-éthyl-3'-pipéridinyl)-1,2-dihydro-3H-dibenzo(deh)isoquinoléine-1,3-dione,
2-[3'-(bis-β-hydroxyéthyl)aminopropyl]-1,2-dihydro-3H-dibenzo(deh)isoquinoléine-1,3-dione,
2-[3'-(diméthylamino)propyl]-1,2-dihydro-3H-dibenzo(deh)isoquinoléine-1,3-dione,
2-(4'-diméthylaminophényl)-1,2-dihydro-3H-dibenzo(deh)isoquinoléine-1,3-dione,
2-[2'-(diméthylamino)éthyl]-1,2-dihydro-11-nitro-3H-dibenzo(deh)isoquinoléine-1,3-dione,
8-amino-2-[2'-(diméthylamino)éthyl]-1,2-dihydro-3H-dibenzo(deh)isoquinoléine-1,3-dione,
11-amino-2-[2'-(diméthylamino)éthyl]-1,2-dihydro-3H-dibenzo(deh)isoquinoléine-1,3-dione,
2-[2'-(diméthylamino)éthyl]-6-éthyl-1,2-dihydro-3H-dibenzo(deh)isoquinoléine-1,3-dione,
7-chloro-2-[2'-(diméthylamino)éthyl]-1,2-dihydro-3H-dibenzo(deh)isoquinoléine-1,3-dione,
2-[2'-(1-pipérazinyl)éthyl]-1,2-dihydro-3H-dibenzo(deh)isoquinoléine-1,3-dione ;
2-[2'-(N-morpholinyl)éthyl]-1,2-dihydro-3H-dibenzo(deh)isoquinoléine-1,3-dione ;
2-[(1'-éthyl-2-pyrrolidinyl)méthyl]-1,2-dihydro-3H-dibenzo(deh)isoquinoléine-1,3-dione ;
2-[2'-(1-méthyl)-2-pyrrolidinyl)éthyl]-1,2-dihydro-3H-dibenzo(deh)isoquinoléine-1,3-dione ;
2-[(3'-pipéridinyl)méthyl]-1,2-dihydro-3H-dibenzo(deh)isoquinoléine-1,3-dione ;
2-(3'-pyridyl)-1,2-dihydro-3H-dibenzo(deh)-isoquinoléine-1,3-dione ;
2-[2'-(2-pyridyl)éthyl]-1,2-dihydro-3H-dibenzo(deh)isoquinoléine-1,3-dione ; et
2-[(1'-aziridinyl)éthyl]-1,2-dihydro-3H-dibenzo-(deh)isoquinoléine-1,3-dione ;

**15.** Composé selon la revendication 1, qui est l'un des composés suivants :

2-[2'-(diméthylamino)éthyl]-1,2-dihydro-3H-dibenzo(deh)isoquinoléine-1,3-dione,
2-[2'-(diméthylamino)éthyl]-1,2-dihydro-8-nitro-3H-dibenzo(deh)isoquinoléine-1,3-dione,
2-[2'-(diméthylamino)éthyl]-1,2-dihydro-6 -éthyl-3H-dibenzo(deh)isoquinoléine-1,3-dione,
10-chloro-2-[2'-(diméthylamino)éthyl]-1,2-dihydro-3H-dibenzo(deh)isoquinoléine-1,3-dione,
2-[2'-(diméthylamino)éthyl]-1,2-dihydro-7-hydroxy-3H-dibenzo(deh)isoquinoléine-1,3-dione,
2-[2'-(diméthylamino)éthyl]-1,2-dihydro-7-méthoxy-3H-dibenzo(deh)isoquinoléine-1,3-dione,
2-[2'-(diméthylamino)éthyl]-1,2-dihydro-7-méthyl-3H-dibenzo(deh)isoquinoléine-1,3-dione,
1,2-dihydro-2-[2'-méthylaminoéthyl]-3H-dibenzo-(deh)isoquinoléine-1,3-dione,
4-acétamido-2-[(2'-diméthylamino)éthyl]-1,2-dihydro-3H-dibenzo(deh)isoquinoléine-1,3-dione,
4-amino-2-[(2'-diméthylamino)éthyl]-1,2-dihydro-3H-dibenzo(deh)isoquinoléine-1,3-dione,
4-hydroxy-2-[(2'-diméthylamino )éthyl]-1,2-dihydro-3H-dibenzo(deh)isoquinoléine-1,3-dione,
4-méthoxy-2-[(2'-diméthylamino)éthyl]-1,2-dihydro-3H-dibenzo(deh)isoquinoléine-1,3-dione,
4-chloro-2-[(2'-diméthylamino)éthyl]-1,2-dihydro-3H-dibenzo(deh)isoquinoléine-1,3-dione,
4-trifluorométhyl-2-[(2'-diméthylamino)éthyl]-1,2-dihydro-3H-dibenzo(deh)isoquinoléine-1,3-dione,
5-acétamido-2-[(2'-diméthylamino)éthyl]-1,2-dihydro-3H-dibenzo(deh)isoquinoléine-1,3-dione,
5-amino-2-[(2'-diméthylamino)éthyl]-1,2-dihydro-3H-dibenzo(deh)isoquinoléine-1,3-dione,
5-méthoxy-2-[(2'-diméthylamino)éthyl]-1,2-dihydro-3H-dibenzo(deh)isoquinoléine-1,3-dione, ou
5-nitro-2-[(2'-diméthylamino)éthyl]-1,2-dihydro-3H-dibenzo(deh)isoquinoléine-1,3-dione.

**16.** Composé selon la revendication 1, qui est l'un des composés suivants :

6-acétamido-2-[(2'-diméthylamino)éthyl]-1,2-dihydro-3H-dibenzo(deh)isoquinoléine-1,3-dione,
6-amino-2-[(2'-diméthylamino)ethyl]-1,2-dihydro-3H-dibenzo(deh)isoquinoléine-1,3-dione,
6-hydroxy-2-[(2'-diméthylamino )éthyl]-1,2-dihydro-3H-dibenzo(deh)isoquinoléine-1,3-dione,
6-méthoxy-2-[(2'-diméthylamino)éthyl]-1,2-dihydro-3H-dibenzo(deh)isoquinoléine-1,3-dione,
6-chloro-2-[(2'-diméthylamino)éthyl]-1,2-dihydro-3H-dibenzo(deh)isoquinoléine-1,3-dione,
6-trifluorométhyl-2-[(2'-diméthylamino)éthyl]-1,2-dihydro-3H-dibenzo(deh)isoquinoléine-1,3-dione,
6-nitro-2-[(2'-diméthylamino)éthyl]-1,2-dihydro-3H-dibenzo(deh)isoquinoléine-1,3-dione.
6-méthyl-2-[(2'-diméthylamino)éthyl]-1,2-dihydro-3H-dibenzo(deh)isoquinoléine-1,3-dione,
7-acétamido-2-[(2'-diméthylamino)éthyl]-1,2-dihydro-3H-dibenzo(deh)isoquinoléine-1,3-dione,
7-amino-2-[(2'-diméthylamino)éthyl]-1,2-dihydro-3H-dibenzo(deh)isoquinoléine-1,3-dione,
7-trifluorométhyl-2-[(2'-diméthylamino)éthyl]-1,2-dihydro-3H-dibenzo(deh)isoquinoléine-1,3-dione,
7-méthylthio-2-[(2'-diméthylamino)éthyl]-1,2-dihydro-3H-dibenzo(deh)isoquinoléine-1,3-dione,
8-acétamido-2-[(2'-diméthylamino)éthyl]-1,2-dihydro-3H-dibenzo(deh)isoquinoléine-1,3-dione,
8-hydroxy-2-[(2'-diméthylamino)éthyl]-1,2-dihydro-3H-dibenzo(deh)isoquinoléine-1,3-dione,
8-méthoxy-2-[(2'-diméthylamino)éthyl]-1,2-dihydro-3H-dibenzo(deh)isoquinoléine-1,3-dione,

8-chloro-2-[(2'-diméthylamino)éthyl]-1,2-dihydro-3H-dibenzo(deh)isoquinoléine-1,3-dione, ou
8-trifluorométhyl-2-[(2'-diméthylamino)éthyl]-1,2-dihydro-3H-dibenzo(deh)isoquinoléine-1,3-dione.

**17.** Composé selon la revendication 1, qui est l'un des composés suivants :

9-acétamido-2-[(2'-diméthylamino)éthyl]-1,2-dihydro-3H-dibenzo(deh)isoquinoléine-1,3-dione,
9-amino-2-[(2'-diméthylamino)éthyl]-1,2-dihydro-3H-dibenzo(deh)isoquinoléine-1,3-dione,
9-hydroxy-2-[(2'-diméthylamino)éthyl]-1,2-dihydro-3H-dibenzo(deh)isoquinoléine-1,3-dione,
9-méthoxy-2-[(2'-diméthylamino)éthyl]-1,2-dihydro-3H-dibenzo(deh)isoquinoléine-1,3-dione,
9-chloro-2-[(2'-diméthylamino)éthyl]-1,2-dihydro-3H-dibenzo(deh)isoquinoléine-1,3-dione,
9-trifluorométhyl-2-[(2'-diméthylamino)éthyl]-1,2-dihydro-3H-dibenzo(deh)isoquinoléine-1,3-dione,
9-nitro-2-[(2'-diméthylamino)éthyl]-1,2-dihydro-3H-dibenzo(deh)isoquinoléine-1,3-dione,
10-acétamido-2-[(2'-diméthylamino)éthyl]-1,2-dihydro-3H-dibenzo(deh)isoquinoléine-1,3-dione,
10-amino-2-[(2'-diméthylamino)éthyl]-1,2-dihydro-3H-dibenzo(deh)isoquinoléine-1,3-dione,
10-hydroxy-2-[(2'-diméthylamino)éthyl]-1,2-dihydro-3H-dibenzo(deh)isoquinoléine-1,3-dione,
10-méthoxy-2-[(2'-diméthylamino)éthyl]-1,2-dihydro-3H-dibenzo(deh)isoquinoléine-1,3-dione,
10-trifluorométhyl-2-[(2'-diméthylamino)éthyl]-1,2-dihydro-3H-dibenzo(deh)isoquinoléine-1,3-dione,
10-nitro-2-[(2'-diméthylamino)éthyl]-1,2-dihydro-3H-dibenzo(deh)isoquinoléine-1,3-dione,
11-acétamido-2-[(2'-diméthylamino)éthyl]-1,2-dihydro-3H-dibenzo(deh)isoquinoléine-1,3-dione,
11-hydroxy-2-[(2'-diméthylamino)éthyl]-1,2-dihydro-3H-dibenzo(deh)isoquinoléine-1,3-dione,
11-méthoxy-2-[(2'-diméthylamino)éthyl]-1,2-dihydro-3H-dibenzo(deh)isoquinoléine-1,3-dione,
2-[(2'-diméthylamino)éthyl]-1,2-dihydro-5-nitro-3H-dibenzo(deh)isoquinoléine-1,3-dione,
5-amino-2-[(2'-diméthylamino)éthyl]-1,2-dihydro-3H-dibenzo(deh)isoquinoléine-1,3-dione, ou
5-acétamido-2-[(2'-diméthylamino)éthyl]-1,2-dihydro-3H-dibenzo(deh)isoquinoléine-1,3-dione.

**18.** Composition pharmaceutique pour le traitement des tumeurs, qui comprend une quantité à effet anti-tumoral d'un composé selon l'une quelconque des revendications 1 à 17, et un excipient pharmaceutique pour ce composé.

**19.** Composé selon l'une quelconque des revendications 1 à 17, pour préparer une composition utile dans le traitement des tumeurs.

**Revendications pour les Etats contractants suivants : ES, GR**

**1.** Procédé pour préparer un composé de formule I :

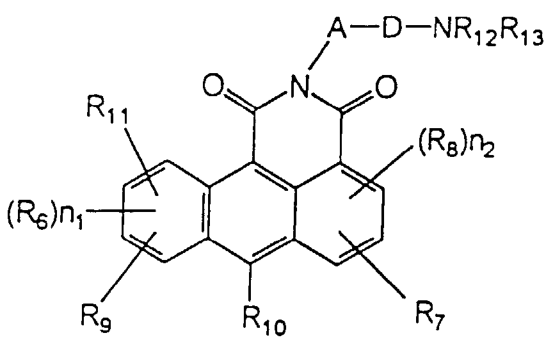

I

dans laquelle

$R_8$, $R_6$ et $R_{10}$ sont chacun indépendamment des autres un atome d'hydrogène, un groupe alkyle en $C_1$-$C_6$, aryle, alcanoyle en $C_2$-$C_7$, formyle, halogéno, nitro, $NR_2R_3$, $OR_1$, ou $SR_1$, hydroxy, méthoxy, cyano, $CO_2H$, $SO_2NR_1R_2$, ou $CONR_1R_2$ ;
$R_1$, $R_2$ et $R_3$ sont chacun indépendamment des autres un atome d'hydrogène, un groupe alkyle en $C_1$-$C_6$, aryl (alkyle en $C_1$-$C_6$), aryle, formyle ou alcanoyle en $C_2$-$C_7$ ;
$R_9$, $R_{11}$, $R_{10}$ et $R_7$ sont chacun indépendamment des autres un atome d'hydrogène ou un groupe alkyle en

$C_1$-$C_6$ ; ou
$R_9$ et $R_{11}$, avec les atomes de carbone auxquels ils sont liés, forment un groupe phényle, ou
$R_9$ et $R_{10}$, avec les atomes de carbone auxquels ils sont liés, forment un groupe phényle, ou
$R_7$ et $R_{10}$, avec les atomes de carbone auxquels ils sont liés, forment un groupe phényle ;
A est $(CR_4R_5)n_3$, un groupe cycloalkyle en $C_3$-$C_{10}$ ou aryle ou une liaison chimique ;
$R_4$ et $R_5$ sont chacun indépendamment de l'autre un atome d'hydrogène ou un groupe alkyle en $C_1$-$C_6$ ;
$R_{12}$ et $R_{13}$ sont chacun indépendamment de l'autre un atome d'hydrogène ou un groupe alkyle en $C_1$-$C_6$ qui est non substitué ou substitué par des substituants hydroxy, mercapto, alcoxy en $C_1$-$C_6$, (alkyle en en $C_2$-$C_7$) carbonyloxy, carbonyle, ou carb(alcoxy inférieur), ou $R_{12}$ et $R_{13}$, avec l'atome d'azote auquel ils sont liés, forment un noyau hétérocyclique ayant de 3 à 6 chaînons ;
D est une liaison chimique, ou encore, avec $NR_{12}$, forme un noyau hétérocyclique à 5 ou 6 chaînons ;
$n_1$ et $n_2$ valent chacun indépendamment de l'autre 0, 1 ou 2 ; et
$n_3$ vaut 0, 1, 2, 3, 4 ou 5,

dans lequel on condense un anhydride anthracène-1,9-dicarboxylique de formule II avec une amine de formule III:

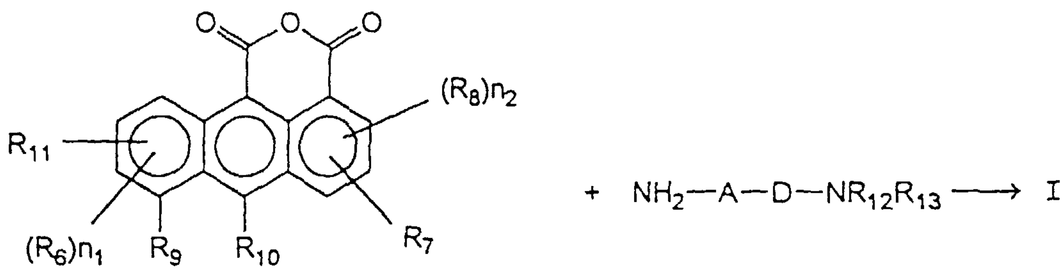

II III

où $R_1$, $R_2$, $R_3$, $R_4$, $R_5$, $R_6$, $R_7$, $R_8$, $R_9$, $R_{10}$, $R_{11}$, $R_{12}$, $R_{13}$, A, D, $n_1$, $n_2$ et $n_3$ sont tels que définis ci-dessus.

**2.** Procédé selon la revendication 1, dans lequel $R_9$, $R_{11}$, $R_{10}$, $R_7$ et $R_8$ sont des atomes d'hydrogène.

**3.** Procédé selon la revendication 1 ou 2, dans lequel $R_6$ est un atome d'hydrogène ou un groupe amino, nitro, hydroxy ou halogéno, et n vaut 1.

**4.** Procédé selon la revendication 3, dans lequel $R_6$ est le groupe 8-nitro, 8-amino, 11-nitro ou 11-amino.

**5.** Procédé selon l'une quelconque des revendications 1 à 4, dans lequel A est un groupe aryle ou $(CR_4R_5)n_3$ et D est une liaison chimique.

**6.** Procédé selon la revendication 5, dans lequel $n_3$ vaut de 2 à 4.

**7.** Procédé selon la revendication 6, dans lequel $R_4$ et $R_5$ sont chacun indépendamment de l'autre un atome d'hydrogène.

**8.** Procédé selon la revendication 1, dans lequel $R_{10}$ est un atome d'hydrogène ou le groupe méthyle, chlore, méthoxy ou hydroxy.

**9.** Procédé selon la revendication 1, dans lequel $R_{12}$ et $R_{13}$ sont des atomes d'hydrogène ou des groupes alkyle en $C_{1\text{-}6}$ non substitués ou substitués par des substituants hydroxy.

**10.** Procédé selon la revendication 1, dans lequel $R_{12}$ et $R_{13}$ sont identiques.

**11.** Procédé selon la revendication 1, dans lequel D, avec $NR_{12}$, forme un noyau azoté ayant 5 ou 6 chaînons.

**12.** Procédé selon la revendication 1, dans lequel $ADNR_{12}R_{13}$ est

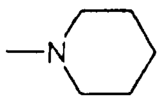 , 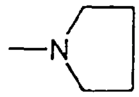 , 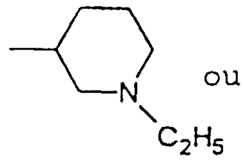 ou 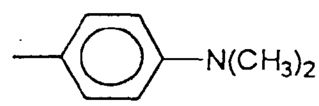

**13.** Procédé selon la revendication 11, dans lequel $R_{12}$ et $R_{13}$, avec l'atome d'azote auquel ils sont liés, forment un noyau pyrrolidine ou pipéridine.

**14.** Procédé selon la revendication 1, dans lequel le composé préparé est l'un des composés suivants :

2-[2'-(N-pyrrolidino)éthyl]-1,2-dihydro-3H-dibenzo(deh)isoquinoléine-1,3-dione,
2-[2'-(N-pipéridino)éthyl]-1,2-dihydro-3H-dibenzo(deh)isoquinoléine-1,3-dione,
2-(1'-éthyl-3'-pipéridinyl)-1,2-dihydro-3H-dibenzo(deh)isoquinoléine-1,3-dione,
2-[3'(bis-β-hydroxyéthyl)aminopropyl]-1,2-dihydro-3H-dibenzo(deh)isoquinoléine-1,3-dione,
2-[3'-(diméthylamino)propyl]-1,2-dihydro-3H-dibenzo(deh)isoquinoléine-1,3-dione,
2-(4'-diméthylaminophényl)-1,2-dihydro-3H-dibenzo(deh)isoquinoléine-1,3-dione,
2-[2'-(diméthylamino)éthyl]-1,2-dihydro-11-nitro-3H-dibenzo(deh)isoquinoléine-1,3-dione,
8-amino-2-[2'-(diméthylamino)éthyl]-1,2-dihydro-3H-dibenzo(deh)isoquinoléine-1,3-dione,
11-amino-2-[2'-(diméthylamino)éthyl]-1,2-dihydro-3H-dibenzo(deh)isoquinoléine-1,3-dione,
2-[2'-(diméthylamino)éthyl]-6-éthyl-1,2-dihydro-3H-dibenzo(deh)isoquinoléine-1,3-dione,
7-chloro-2-[2'-(diméthylamino)éthyl]-1,2-dihydro-3H-dibenzo(deh)isoquinoléine-1,3-dione,
2-[2'-(1-pipérazinyl)éthyl]-1,2-dihydro-3H-dibenzo(deh)isoquinoléine-1,3-dione ;
2-[2'-(N-morpholinyl)éthyl]-1,2-dihydro-3H-dibenzo(deh)isoquinoléine-1,3-dione ;
2-[(1'-éthyl-2-pyrrolidinyl)méthyl]-1,2-dihydro-3H-dibenzo(deh)isoquinoléine-1,3-dione ;
2-[2'-(1-méthyl)-2-pyrrolidinyl)éthyl]-1,2-dihydro-3H-dibenzo(deh)isoquinoléine-1,3-dione ;
2-[(3'-pipéridinyl)méthyl]-1,2-dihydro-3H-dibenzo(deh)isoquinoléine-1,3-dione ;
2-[(3'-pyridyl)-1,2-dihydro-3H-dibenzo(deh)-isoquinoléine-1,3-dione ;
2-[2'-(2-pyridyl)éthyl]-1,2-dihydro-3H-dibenzo-(deh)isoquinoléine-1,3-dione ; et
2-[(1'-aziridinyl)éthyl]-1,2-dihydro-3H-dibenzo-((deh)isoquinoléine-1,3-dione ;

**15.** Procédé selon la revendication 1, dans lequel le composé préparé est l'un des composés suivants :

2-[2'-(diméthylamino)éthyl]-1,2-dihydro-3H-dibenzo(deh)isoquinoléine-1,3-dione,
2-[2'-(diméthylamino)éthyl]-1,2-dihydro-8-nitro-3H-dibenzo(deh)isoquinoléine-1,3-dione,
2-[2'-(diméthylamino)éthyl]-1,2-dihydro-6-éthyl-3H-dibenzo(deh)isoquinoléine-1,3-dione,
10-chloro-2-[2'-(diméthylamino)éthyl]-1,2-dihydro-3H-dibenzo(deh)isoquinoléine-1,3-dione,
2-[2'-(diméthylamino)éthyl]-1,2-dihydro-7-hydroxy-3H-dibenzo(deh)isoquinoléine-1,3-dione,
2-[2'-(diméthylamino)éthyl]-1,2-dihydro-7-méthoxy-3H-dibenzo(deh)isoquinoléine-1,3-dione,
2-[2'-(diméthylamino)éthyl]-1,2-dihydro-7-méthyl-3H-dibenzo(deh)isoquinoléine-1,3-dione,
1,2-dihydro-2-[2'-méthylaminoéthyl]-3H-dibenzo(deh)isoquinoléine-1,3-dione,
4-acétamido-2-[(2'-diméthylamino)éthyl]-1,2-dihydro-3H-dibenzo(deh)isoquinoléine-1,3-dione,
4-amino-2-[(2'-diméthylamino)éthyl]-1,2-dihydro-3H-dibenzo(deh)isoquinoléine-1,3-dione,
4-hydroxy-2-[(2'-diméthylamino )éthyl]-1,2-dihydro-3H-dibenzo(deh)isoquinoléine-1,3-dione,
4-méthoxy-2-[(2'-diméthylamino)éthyl]-1,2-dihydro-3H-dibenzo(deh)isoquinoléine-1,3-dione,
4-chloro-2-[(2'-diméthylamino)éthyl]-1,2-dihydro-3H-dibenzo(deh)isoquinoléine-1,3-dione,
4-trifluorométhyl-2-[(2'-diméthylamino)éthyl]-1,2-dihydro-3H-dibenzo(deh)isoquinoléine-1,3-dione,
5-acétamido-2-[(2'-diméthylamino)éthyl]-1,2-dihydro-3H-dibenzo(deh)isoquinoléine-1,3-dione,
5-amino-2-[(2'-diméthylamino)éthyl]-1,2-dihydro-3H-dibenzo(deh)isoquinoléine-1,3-dione,
5-méthoxy-2-[(2'-diméthylamino)éthyl]-1,2-dihydro-3H-dibenzo(deh)isoquinoléine-1,3-dione, ou
5-nitro-2-[(2'-diméthylamino)éthyl]-1,2-dihydro-3H-dibenzo(deh)isoquinoléine-1,3-dione.

**16.** Procédé selon la revendication 1, dans lequel le composé préparé est l'un des composés suivants :

6-acétamido-2-[(2'-diméthylamino)éthyl]-1,2-dihydro-3H-dibenzo(deh)isoquinoléine-1,3-dione,
6-amino-2-[(2'-diméthylamino)éthyl]-1,2-dihydro-3H-dibenzo(deh)isoquinoléine-1,3-dione,
6-hydroxy-2-[(2'-diméthylamino)éthyl]-1,2-dihydro-3H-dibenzo(deh)isoquinoléine-1,3-dione,
6-méthoxy-2-[(2'-diméthylamino)éthyl]-1,2-dihydro-3H-dibenzo(deh)isoquinoléine-1,3-dione,
6-chloro-2-[(2'-diméthylamino)éthyl]-12-dihydro-3H-dibenzo(deh)isoquinoléine-1,3-dione,
6-trifluorométhyl-2-[(2'-diméthylamino)éthyl]-1,2-dihydro-3H-dibenzo(deh)isoquinoléine-1,3-dione,
6-nitro-2-[(2'-diméthylamino)éthyl]-1,2-dihydro-3H-dibenzo(deh)isoquinoléine-1,3-dione.
6-méthyl-2-[(2'-diméthylamino)éthyl]-1,2-dihydro-3H-dibenzo(deh)isoquinoléine-1,3-dione,
7-acétamido-2-[(2'-diméthylamino)éthyl]-1,2-dihydro-3H-dibenzo(deh)isoquinoléine-1,3-dione,
7-amino-2-[(2'-diméthylamino)éthyl]-1,2-dihydro-3H-dibenzo(deh)isoquinoléine-1,3-dione,
7-trifluorométhyl-2-[(2'-diméthylamino)éthyl]-1,2-dihydro-3H-dibenzo(deh)isoquinoléine-1,3-dione,
7-méthylthio-2-[(2'-diméthylamino)éthyl]-1,2-dihydro-3H-dibenzo(deh)isoquinoléine-1,3-dione,
8-acétamido-2-[(2'-diméthylamino)éthyl]-1,2-dihydro-3H-dibenzo(deh)isoquinoléine-1,3-dione,
8-hydroxy-2-[(2'-diméthylamino)éthyl]-1,2-dihydro-3H-dibenzo(deh)isoquinoléine-1,3-dione,
8-méthoxy-2-[(2'-diméthylamino)éthyl]-1,2-dihydro-3H-dibenzo(deh)isoquinoléine-1,3-dione,
8-chloro-2-[(2'-diméthylamino)éthyl]-1,2-dihydro-3H-dibenzo(deh)isoquinoléine-1,3-dione, ou
8-trifluorométhyl-2-[(2'-diméthylamino)éthyl]-1,2-dihydro-3H-dibenzo(deh)isoquinoléine-1,3-dione.

**17.** Procédé selon la revendication 1, dans lequel le composé préparé est l'un des composés suivants :

9-acétamido-2-[(2'-diméthylamino)éthyl]-1,2-dihydro-3H-dibenzo(deh)isoquinoléine-1,3-dione,
9-amino-2-[(2-diméthylamino)éthyl]-1,2-dihydro-3H-dibenzo(deh)isoquinoléine-1,3-dione,
9-hydroxy-2-[(2'-diméthylamino)éthyl]-1,2-dihydro-3H-dibenzo(deh)isoquinoléine-1,3-dione,
9-méthoxy-2-[(2'-diméthylamino)éthyl]-1,2-dihydro-3H-dibenzo(deh)isoquinoléine-1,3-dione,
9-chloro-2-[(2'-diméthylamino)éthyl]-1,2-dihydro-3H-dibenzo(deh)isoquinoléine-1,3-dione,
9-trifluorométhyl-2-[(2'-diméthylamino)éthyl]-1,2-dihydro-3H-dibenzo(deh)isoquinoléine-1,3-dione,
9-nitro-2-[(2'-diméthylamino)éthyl]-1,2-dihydro-3H-dibenzo(deh)isoquinoléine-1,3-dione,
10-acétamido-2-[(2'-diméthylamino)éthyl]-1,2-dihydro-3H-dibenzo(deh)isoquinoléine-1,3-dione,
10-amino-2-[(2'-diméthylamino)éthyl]-1,2-dihydro-3H-dibenzo(deh)isoquinoléine-1,3-dione,
10-hydroxy-2-[(2'-diméthylamino)éthyl]-1,2-dihydro-3H-dibenzo(deh)isoquinoléine-1,3-dione,
10-méthoxy-2-[(2'-diméthylamino)éthyl]-1,2-dihydro-3H-dibenzo(deh)isoquinoléine-1,3-dione,
10-trifluorométhyl-2-[(2'-diméthylamino)éthyl]-1,2-dihydro-3H-dibenzo(deh)isoquinoléine-1,3-dione,
10-nitro-2-[(2'-diméthylamino)éthyl]-1,2-dihydro-3H-dibenzo(deh)isoquinoléine-1,3-dione,
11-acétamido-2-[(2'-diméthylamino)éthyl]-1,2-dihydro-3H-dibenzo(deh)isoquinoléine-1,3-dione,
11-hydroxy-2-[(2'-diméthylamino)éthyl]-1,2-dihydro-3H-dibenzo(deh)isoquinoléine-1,3-dione,
11-méthoxy-2-[(2'-diméthylamino)éthyl]-1,2-dihydro-3H-dibenzo(deh)isoquinoléine-1,3-dione,
2-[(2'-diméthylamino)éthyl]-1,2-dihydro-5-nitro-3H-dibenzo(deh)isoquinoléine-1,3-dione,
5-amino-2-[(2'-diméthylamino)éthyl]-1,2-dihydro-3H-dibenzo(deh)isoquinoléine-1,3-dione, ou
5-acétamido-2-[(2'-diméthylamino)éthyl]-1,2-dihydro-3H-dibenzo(deh)isoquinoléine-1,3-dione.

**18.** Procédé pour préparer une composition pharmaceutique, qui comprend l'étape consistant à mélanger une quantité à effet anti-tumoral d'un composé préparé selon l'une quelconque des revendications 1 à 17, avec un excipient pharmaceutique pour ce composé.

**19.** Composé selon l'une quelconque des revendications 1 à 17, pour préparer une composition utile dans le traitement des tumeurs.